# NEW EUROPEAN PATENT SPECIFICATION

(11) **EP 1 962 895 B2**
(45) Date of publication and mention of the opposition decision: **20.01.2016**
(45) Mention of the grant of the patent: 13.02.2013
(21) Application number: 06848586.1
(22) Date of filing: 15.12.2006
(51) Int. Cl.: A61K 39/00

(54) **THERAPEUTIC USE OF A Dll4 ANTAGONIST AND A VEGF INHIBITOR FOR INHIBITING TUMOR GROWTH**
THERAPEUTISCHE VERWENDUNG EINES Dll4 ANTAGONISTEN UND EINES VEGF INHIBITORS ZUR HEMMUNG VON TUMORWACHSTUM
UTILISATION THERAPEUTIQUES D'UN ANTAGONIST Dll4 ET D'UN INHIBITEUR VEGF POUR L'INHIBITION DE CROISSANCE TUMORALE

(30) Priority: 16.12.2005 US 751173 P; 08.02.2006 US 771276 P; 31.03.2006 US 788456 P; 12.07.2006 US 830543 P
(43) Date of publication of application: 03.09.2008
(73) Proprietor: REGENERON PHARMACEUTICALS, INC., Tarrytown, NY 10591 (US)
(72) Inventor: NOGUERA, Irene, Bay Shore, NY 11706 (US); THURSTON, Gavin, White Plains, NY 10603 (US); GALE, Nicholas, Yorktown Heights, NY 10598 (US); SMITH, Eric, New York, NY 10016 (US)
(74) Representative: Bentham, Andrew
(86) International application number: PCT/US2006/047848
(87) International publication number: WO 2007/070671

(56) References cited:
- WO-A1-00/75319
- WO-A2-98/45331
- WO-A2-03/042246
- WO-A2-03/050502
- WO-A2-2008/060705
- US-A1- 2002 119 153
- US-A1- 2006 134 121
- PATEL NILAY S ET AL: "Up-regulation of delta-like 4 ligand in human tumor vasculature and the role of basal expression in endothelial cell function" CANCER RESEARCH, vol. 65, no. 19, October 2005 (2005-10), pages 8690-8697,8679, XP002452160 ISSN: 0008-5472
- GALE NICHOLAS W ET AL: "Haploinsufficiency of delta-like 4 ligand results in embryonic lethality due to major defects in arterial and vascular development" PROCEEDINGS OF THE NATIONAL ACADEMY OF SCIENCES OF USA, NATIONAL ACADEMY OF SCIENCE, WASHINGTON, DC, US, vol. 101, no. 45, 9 November 2004 (2004-11-09), pages 15949-15954, XP002432725 ISSN: 0027-8424
- NOGUERA IRENE ET AL: "Expression of Delta-like 4 (Dll4) ligand in mouse tumor models" PROCEEDINGS OF THE ANNUAL MEETING OF THE AMERICAN ASSOCIATION FOR CANCER RESEARCH, NEW YORK, NY, US, vol. 46, no. Suppl S, April 2005 (2005-04), page 1104, XP001245667 ISSN: 0197-016X
- MAILHOS CAROLINA ET AL: "Delta4, an endothelial specific Notch ligand expressed at sites of physiological and tumor angiogenesis" DIFFERENTIATION, vol. 69, no. 2-3, December 2001 (2001-12), pages 135-144, XP002452161 ISSN: 0301-4681
- NOGUERA-TROISE IRENE ET AL: "Blockade of Dll4 inhibits tumour growth by promoting non-productive angiogenesis" NATURE (LONDON), vol. 444, no. 7122, December 2006 (2006-12), pages 1032-1037, XP002452162 ISSN: 0028-0836
- RIDGWAY JOHN ET AL: "Inhibition of Dll4 signalling inhibits tumour growth by deregulating angiogenesis" NATURE (LONDON), vol. 444, no. 7122, December 2006 (2006-12), pages 1083-1087, XP002452163 ISSN: 0028-0836
- US 62365804 P 29 October 2004
- HOLASH J. ET AL: 'VEGF-Trap: A VEGF blocker with potent antitumor effects' PNAS vol. 99, no. 17, 20 August 2002, pages 11393 - 11398
- ZHAO-JUN LIU ET AL: 'Regulation of Notch1 and D114 by Vascular Endothelial Growth Factor in Arterial Endothelial Cells: Implications for Modulating Arteriogenesis and Angiogenesis' MOLECULAR AND CELLULAR BIOLOGY vol. 23, no. 1, January 2003, pages 14 - 25
- JI-LIANG LI ET AL.: 'Notch signaling from tumor cells: A new mechanism of angiogenesis' CANCER CELL vol. 8, July 2005, pages 1 - 3
- CASSIN KIMMEL WILLIAMS ET AL.: 'Up-regulation of the Notch ligand Delta-like 4 inhibits VEGF-induced endothelialcell function' BLOOD vol. 107, no. 3, 01 February 2006, pages 931 - 939
- US 81134906 P 06 June 2006
- LU K.V. ET AL: 'Mechanisms of evasive resistance to anti-VEGF therapy in glioblastoma' CNS ONCOL vol. 2, no. 1, January 2013, pages 49 - 65
- TON N.C. ET AL: 'Resistance to Anti-VEGF Agents' CURRENT PHARMACEUTICAL DESIGN vol. 10, 2004, pages 51 - 64
- KIM E.S. ET AL: 'Potent VEGF blockade causes regression of coopted vessels in a model of neuroblastoma' PNAS vol. 99, no. 17, 20 August 2002, pages 11399 - 11404
- PISANO C. ET AL: 'Undersulfated, low-molecular-weight glycol-split heparin as an antiangiogenic VEGF antagonist' GLYCOBIOLOGY vol. 15, no. 2, October 2004, pages 1C - 6C
- GAGNON M.L. ET AL: 'Identification of a natural soluble neuropilin-1 that binds vascular endothelialgrowth factor: In vivo expression and antitumor activity' PNAS vol. 97, no. 6, 14 March 2000, pages 2573 - 2578
- KUO C.J. ET AL: 'Comparative evaluation of the antitumor activity of antiangiogenic proteins delivered by gene transfer' PNAS vol. 98, no. 8, 10 April 2001, pages 4605 - 4610

### SEQUENCE LISTING

<110> Regeneron Pharmaceuticals, Inc
<120> Therapeutic Methods for Inhibiting Tumour Growth with DLL4 Antagonists
<130> 2071A-WO
<140> To be assigned
   <141> 2006-12-15
<150> 60/751, 176
   <151> 2005-12-16
<150> 60/771,276
   <151> 2006-02-08
<150> 60/788, 456
   <151> 2006-03-31
<150> 60/830, 543
   <151> 2006-07-12
<160> 26
<170> FastSEQ for windows version 4.0
<210> 1
   <211> 2058
   <212> DNA
   <213> Homo sapiens
<400> 1
   atggcggcag cgtcccggag cgcctctggc tgggcgctac tgctgctggt ggcactttgg 60
   cagcagcgcg cggccggctc cggcgtcttc cagctgcagc tgcaggagtt catcaacgag 120
   cgcggcgtac tggccagtgg gcggccttgc gagcccggct gccggacttt cttccgcgtc 180
   tgccttaagc acttccaggc ggtcgtctcg cccggaccct gcaccttcgg gaccgtctcc 240
   acgccggtat tgggcaccaa ctccttcgct gtccgggacg acagtagcgg cggggggcgc 300
   aaccctctcc aactgccctt caatttcacc tggccgggta ccttctcgct catcatcgaa 360
   gcttggcacg cgccaggaga cgacctgcgg ccagaggcct tgccaccaga tgcactcatc 420
   agcaagatcg tcatccaggg ctccctagct gtgggtcaga actggttatt ggatgagcaa 480
   accagcaccc tcacaaggct gcgctactct taccgggtca tctgcagtga caactactat 540
   ggagacaact gctcccgcct gtgcaagaag cgcaatgacc acttcggcca ctatgtgtgc 600
   cagccagatg gcaacttgtc ctgcctgccc ggttggactg gggaatattg ccaacagcct 660
   atctgtcttt cgggctgtca tgaacagaat ggctactgca gcaagccagc agagtgcctc 720
   tgccgcccag gctggcaggg ccggctgtgt aacgaatgca tcccccacaa tggctgtcgc 780
   cacggcacct gcagcactcc ctggcaatgt acttgtgatg agggctgggg aggcctgttt 840
   tgtgaccaag atctcaacta ctgcacccac cactccccat gcaagaatgg ggcaacgtgc 900
   tccaacagtg ggcagcgaag ctacacctgc acctgtcgcc caggctacac tggtgtggac 960
   tgtgagctgg agctcagcga gtgtgacagc aacccctgtc gcaatggagg cagctgtaag 1020
   gaccaggagg atggctacca ctgcctgtgt cctccgggct actatggcct gcattgtgaa 1080
   cacagcacct tgagctgcgc cgactccccc tgcttcaatg ggggctcctg ccgggagcgc 1140
   aaccaggggg ccaactatgc ttgtgaatgt ccccccaact tcaccggctc caactgcgag 1200
   aagaaagtgg acaggtgcac cagcaacccc tgtgccaacg ggggacagtg cctgaaccga 1260
   ggtccaagcc gcatgtgccg ctgccgtcct ggattcacgg gcacctactg tgaactccac 1320
   gtcagcgact gtgcccgtaa cccttgcgcc cacggtggca cttgccatga cctggagaat 1380
   gggctcatgt gcacctgccc tgccggcttc tctggccgac gctgtgaggt gcggacatcc 1440
   atcgatgcct gtgcctcgag tccctgcttc aacagggcca cctgctacac cgacctctcc 1500
   acagacacct ttgtgtgcaa ctgcccttat ggctttgtgg gcagccgctg cgagttcccc 1560
   gtgggcttgc cgcccagctt cccctgggtg gccgtctcgc tgggtgtggg gctggcagtg 1620
   ctgctggtac tgctgggcat ggtggcagtg gctgtgcggc agctgcggct tcgacggccg 1680
   gacgacggca gcagggaagc catgaacaac ttgtcggact tccagaagga caacctgatt 1740
   cctgccgccc agcttaaaaa cacaaaccag aagaaggagc tggaagtgga ctgtggcctg 1800
   gacaagtcca actgtggcaa acagcaaaac cacacattgg actataatct ggccccaggg 1860
   cccctggggc gggggaccat gccaggaaag tttccccaca gtgacaagag cttaggagag 1920
   aaggcgccac tgcggttaca cagtgaaaag ccagagtgtc ggatatcagc gatatgctcc 1980
   cccagggact ccatgtacca gtctgtgtgt ttgatatcag aggagaggaa tgaatgtgtc 2040
   attgccacgg aggtataa 2058
<210> 2
   <211> 685
   <212> PRT
   <213> Homo sapiens
<400> 2
<210> 3
   <211> 3427
   <212> DNA
   <213> Mus musculs
<400> 3
   ctcgcaggct aggaacccga ggccaagagc tgcagccaaa gtcacttggg tgcagtgtac 60
   tccctcacta gcccgctcga gaccctagga tttgctccag gacacgtact tagagcagcc 120
   accgcccagt cgccctcacc tggattacct accgaggcat cgagcagcgg agtttttgag 180
   aaggcgacaa gggagcagcg tcccgagggg aatcagcttt tcaggaactc ggctggcaga 240
   cgggacttgc gggagagcga catccctaac aagcagattc ggagtcccgg agtggagagg 300
   acaccccaag ggatgacgcc tgcgtcccgg agcgcctgtc gctgggcgct actgctgctg 360
   gcggtactgt ggccgcagca gcgcgctgcg ggctccggca tcttccagct gcggctgcag 420
   gagttcgtca accagcgcgg tatgctggcc aatgggcagt cctgcgaacc gggctgccgg 480
   actttcttcc gcatttgcct taagcacttc caggcaacct tctccgaggg accctgcacc 540
   tttggcaatg tctccacgcc ggtattgggc accaactcct tcgtcgtcag ggacaagaat 600
   agcggcagtg gtcgcaaccc tctgcagttg cccttcaatt tcacctggcc gggaaccttc 660
   tcactcaaca tccaagcttg gcacacaccg ggagacgacc tgcggccaga gacttcgcca 720
   ggaaactctc tcatcagcca aatcatcatc caaggctctc ttgctgtggg taagatttgg 780
   cgaacagacg agcaaaatga caccctcacc agactgagct actcttaccg ggtcatctgc 840
   agtgacaact actatggaga gagctgttct cgcctatgca agaagcgcga tgaccacttc 900
   ggacattatg agtgccagcc agatggcagc ctgtcctgcc tgccgggctg gactgggaag 960
   tactgtgacc agcctatatg tctttctggc tgtcatgagc agaatggtta ctgcagcaag 1020
   ccagatgagt gcatctgccg tccaggttgg cagggtcgcc tgtgcaatga atgtatcccc 1080
   cacaatggct gtcgtcatgg cacctgcagc atcccctggc agtgtgcctg cgatgaggga 1140
   tggggaggtc tgttttgtga ccaagatctc aactactgta ctcaccactc tccgtgcaag 1200
   aatggatcaa cgtgttccaa cagtgggcca aagggttata cctgcacctg tctcccaggc 1260
   tacactggtg agcactgtga gctgggactc agcaagtgtg ccagcaaccc ctgtcgaaat 1320
   ggtggcagct gtaaggacca ggagaatagc taccactgcc tgtgtccccc aggctactat 1380
   ggccagcact gtgagcatag taccttgacc tgtgcggact caccctgctt caatgggggc 1440
   tcttgccggg agcgcaacca ggggtccagt tatgcctgcg aatgcccccc caactttacc 1500
   ggctctaact gtgagaagaa agtagacagg tgtaccagca acccgtgtgc caatggaggc 1560
   cagtgcctga acagaggtcc aagccgaacc tgccgctgcc ggcctggatt cacaggcacc 1620
   cactgtgaac tgcacatcag cgattgtgcc cgaagtccct gtgcccacgg gggcacttgc 1680
   cacgatctgg agaatggccc tgtgtgcacc tgccccgctg gcttctctgg caggcgctgc 1740
   gaggtgcgga taacccacga tgcctgtgcc tccggaccct gcttcaatgg ggccacctgc 1800
   tacactggcc tctccccaaa caacttcgtc tgcaactgtc cttatggctt tgtgggcagc 1860
   cgctgcgagt ttcccgtggg cttgccaccc agcttcccct gggtagctgt ctcgctgggc 1920
   gtggggctag tggtactgct ggtgctgctg gtcatggtgg tagtggctgt gcggcagctg 1980
   cggcttcgga ggcccgatga cgagagcagg gaagccatga acaatctgtc agacttccag 2040
   aaggacaacc taatccctgc cgcccagctc aaaaacacaa accagaagaa ggagctggaa 2100
   gtggactgtg gtctggacaa gtccaattgt ggcaaactgc agaaccacac attggactac 2160
   aatctagccc cgggactcct aggacggggc agcatgcctg ggaagtatcc tcacagtgac 2220
   aagagcttag gagagaaggt gccacttcgg ttacacagtg agaagccaga gtgtcgaata 2280
   tcagccattt gctctcccag ggactctatg taccaatcag tgtgtttgat atcagaagag 2340
   aggaacgagt gtgtgattgc cacagaggta taaggcagga gcctactcag acacccagct 2400
   ccggcccagc agctgggcct tccttctgca ttgtttacat tgcatcctgt atgggacatc 2460
   tttagtatgc acagtgctgc tctgcggagg aggagggaat ggcatgaact gaacagactg 2520
   tgaacccgcc aagagttgca ccggctctgc acacctccag gagtctgcct ggcttcagat 2580
   gggcagcccc gccaagggaa cagagttgag gagttagagg agcatcagtt gagctgatat 2640
   ctaaggtgcc tctcgaactt ggacttgctc tgccaacagt ggtcatcatg gagctcttga 2700
   ctgttctcca gagagtggca gtggccctag tgggtcttgg cgctgctgta gctcctgtgg 2760
   gcatctgtat ttccaaagtg cctttgccca gactccatcc tcacagctgg gcccaaatga 2820
   gaaagcagag aggaggcttg caaaggatag gcctcccgca ggcagaacag ccttggagtt 2880
   tggcattaag caggagctac tctgcaggtg aggaaagccc gaggagggga cacgtgtgac 2940
   tcctgcctcc aaccccagca ggtggggtgc cacctgcagc ctctaggcaa gagttggtcc 3000
   ttcccctggt cctggtgcct ctgggctcat gtgaacagat gggcttaggg cacgcccctt 3060
   ttgccagcca ggggtacagg cctcactggg gagctcaggg ccttcatgct aaactcccaa 3120
   taagggagat ggggggaagg gggctgtggc ctaggccctt ccctccctca cacccatttt 3180
   tgggcccttg agcctgggct ccaccagtgc ccactgttgc cccgagacca accttgaagc 3240
   cgattttcaa aaatcaataa tatgaggttt tgttttgtag tttattttgg aatctagtat 3300
   tttgataatt taagaatcag aagcactggc ctttctacat tttataacat tattttgtat 3360
   ataatgtgta tttataatat gaaacagatg tgtacataaa aaaaaaaaaa aaaaaaaaaa 3420
   aaaaaaa                                                           3427
<210> 4
   <211> 686
   <212> PRT
   <213> Mus musculus
<400> 4
   Met Thr Pro Ala Ser Arg Ser Ala Cys Arg Trp Ala Leu Leu Leu Leu
   1               5                   10                  15
   Ala Val Leu Trp Pro Gln Gln Arg Ala Ala Gly Ser Gly Ile Phe Gln
               20                  25                  30
   Leu Arg Leu Gln Glu Phe Val Asn Gln Arg Gly Met Leu Ala Asn Gly
           35                  40                  45
   Gln Ser Cys Glu Pro Gly Cys Arg Thr Phe Phe Arg Ile Cys Leu Lys
       50                  55                  60
   His Phe Gln Ala Thr Phe Ser Glu Gly Pro Cys Thr Phe Gly Asn Val
   65 70 75 80
   Ser Thr Pro Val Leu Gly Thr Asn Ser Phe Val Val Arg Asp Lys Asn
   85 90 95
   Ser Gly Ser Gly Arg Asn Pro Leu Gln Leu Pro Phe Asn Phe Thr Trp
   100 105 110
   Pro Gly Thr Phe Ser Leu Asn Ile Gln Ala Trp His Thr Pro Gly Asp
   115 120 125
   Asp Leu Arg Pro Glu Thr Ser Pro Gly Asn Ser Leu Ile Ser Gln Ile
   130 135 140
   Ile Ile Gln Gly Ser Leu Ala Val Gly Lys Ile Trp Arg Thr Asp Glu
   145 150 155 160
   Gln Asn Asp Thr Leu Thr Arg Leu Ser Tyr Ser Tyr Arg Val Ile Cys
   165 170 175
   Ser Asp Asn Tyr Tyr Gly Glu Ser Cys Ser Arg Leu Cys Lys Lys Arg
   180 185 190
   Asp Asp His Phe Gly His Tyr Glu Cys Gln Pro Asp Gly Ser Leu Ser
   195 200 205
   Cys Leu Pro Gly Trp Thr Gly Lys Tyr Cys Asp Gln Pro Ile Cys Leu
   210 215 220
   Ser Gly Cys His Glu Gln Asn Gly Tyr Cys Ser Lys Pro Asp Glu Cys
   225 230 235 240
   Ile Cys Arg Pro Gly Trp Gln Gly Arg Leu Cys Asn Glu Cys Ile Pro
   245 250 255
   His Asn Gly Cys Arg His Gly Thr Cys Ser Ile Pro Trp Gln Cys Ala
   260 265 270
   Cys Asp Glu Gly Trp Gly Gly Leu Phe Cys Asp Gln Asp Leu Asn Tyr
   275 280 285
   Cys Thr His His Ser Pro Cys Lys Asn Gly Ser Thr Cys Ser Asn Ser
   290 295 300
   Gly Pro Lys Gly Tyr Thr Cys Thr Cys Leu Pro Gly Tyr Thr Gly Glu
   305 310 315 320
   His Cys Glu Leu Gly Leu Ser Lys Cys Ala Ser Asn Pro Cys Arg Asn
   325 330 335
   Gly Gly Ser Cys Lys Asp Gln Glu Asn Ser Tyr His Cys Leu Cys Pro
   340 345 350
   Pro Gly Tyr Tyr Gly Gln His Cys Glu His Ser Thr Leu Thr Cys Ala
   355 360 365
   Asp Ser Pro Cys Phe Asn Gly Gly Ser Cys Arg Glu Arg Asn Gln Gly
   370 375 380
   Ser Ser Tyr Ala Cys Glu Cys Pro Pro Asn Phe Thr Gly Ser Asn Cys
   385 390 395 400
   Glu Lys Lys Val Asp Arg Cys Thr Ser Asn Pro Cys Ala Asn Gly Gly
   405 410 415
   Gln Cys Leu Asn Arg Gly Pro Ser Arg Thr Cys Arg Cys Arg Pro Gly
   420 425 430
   Phe Thr Gly Thr His Cys Glu Leu His Ile Ser Asp Cys Ala Arg Ser
   435 440 445
   Pro Cys Ala His Gly Gly Thr Cys His Asp Leu Glu Asn Gly Pro Val
   450 455 460
   Cys Thr Cys Pro Ala Gly Phe Ser Gly Arg Arg Cys Glu Val Arg Ile
   465 470 475 480
   Thr His Asp Ala Cys Ala Ser Gly Pro Cys Phe Asn Gly Ala Thr Cys
   485 490 495
   Tyr Thr Gly Leu Ser Pro Asn Asn Phe Val Cys Asn Cys Pro Tyr Gly
   500 505 510
   Phe Val Gly Ser Arg Cys Glu Phe Pro Val Gly Leu Pro Pro Ser Phe
   515 520 525
   Pro Trp Val Ala Val Ser Leu Gly Val Gly Leu Val Val Leu Leu Val
   530 535 540
   Leu Leu Val Met Val Val Val Ala Val Arg Gln Leu Arg Leu Arg Arg
   545 550 555 560
   Pro Asp Asp Glu Ser Arg Glu Ala Met Asn Asn Leu Ser Asp Phe Gln
   565 570 575
   Lys Asp Asn Leu Ile Pro Ala Ala Gln Leu Lys Asn Thr Asn Gln Lys
   580 585 590
   Lys Glu Leu Glu Val Asp Cys Gly Leu Asp Lys Ser Asn Cys Gly Lys
   595 600 605
   Leu Gln Asn His Thr Leu Asp Tyr Asn Leu Ala Pro Gly Leu Leu Gly
   610 615 620
   Arg Gly Ser Met Pro Gly Lys Tyr Pro His Ser Asp Lys Ser Leu Gly
   625 630 635 640
   Glu Lys Val Pro Leu Arg Leu His Ser Glu Lys Pro Glu Cys Arg Ile
   645 650 655
   Ser Ala Ile Cys Ser Pro Arg Asp Ser Met Tyr Gln Ser Val Cys Leu
   660 665 670
   Ile Ser Glu Glu Arg Asn Glu Cys Val Ile Ala Thr Glu Val
   675 680 685
<210> 5
   <211> 9312
   <212> DNA
   <213> Homo sapien
<400> 5
<210> 6
   <211> 2556
   <212> PRT
   <213> Homo sapien
<400> 6
   Met Pro Pro Leu Leu Ala Pro Leu Leu Cys Leu Ala Leu Leu Pro Ala
   1 5 10 15
   Leu Ala Ala Arg Gly Pro Arg Cys Ser Gln Pro Gly Glu Thr Cys Leu
   20 25 30
   Asn Gly Gly Lys Cys Glu Ala Ala Asn Gly Thr Glu Ala Cys Val Cys
   35 40 45
   Gly Gly Ala Phe Val Gly Pro Arg Cys Gln Asp Pro Asn Pro Cys Leu
   50 55 60
   Ser Thr Pro Cys Lys Asn Ala Gly Thr Cys His Val Val Asp Arg Arg
   65 70 75 80
   Gly Val Ala Asp Tyr Ala Cys Ser Cys Ala Leu Gly Phe Ser Gly Pro
   85 90 95
   Leu Cys Leu Thr Pro Leu Asp Asn Ala Cys Leu Thr Asn Pro Cys Arg
   100 105 110
   Asn Gly Gly Thr Cys Asp Leu Leu Thr Leu Thr Glu Tyr Lys Cys Arg
   115 120 125
   Cys Pro Pro Gly Trp Ser Gly Lys Ser Cys Gln Gln Ala Asp Pro Cys
   130 135 140
   Ala Ser Asn Pro Cys Ala Asn Gly Gly Gln Cys Leu Pro Phe Glu Ala
   145 150 155 160
   Ser Tyr Ile Cys His Cys Pro Pro Ser Phe His Gly Pro Thr Cys Arg
   165 170 175
   Gln Asp Val Asn Glu Cys Gly Gln Lys Pro Gly Leu Cys Arg His Gly
   180 185 190
   Gly Thr Cys His Asn Glu Val Gly Ser Tyr Arg Cys Val Cys Arg Ala
   195 200 205
   Thr His Thr Gly Pro Asn Cys Glu Arg Pro Tyr Val Pro Cys Ser Pro
   210 215 220
   Ser Pro Cys Gln Asn Gly Gly Thr Cys Arg Pro Thr Gly Asp Val Thr
   225 230 235 240
   His Glu Cys Ala Cys Leu Pro Gly Phe Thr Gly Gln Asn Cys Glu Glu
   245 250 255
   Asn Ile Asp Asp Cys Pro Gly Asn Asn Cys Lys Asn Gly Gly Ala Cys
   260 265 270
   Val Asp Gly Val Asn Thr Tyr Asn Cys Arg Cys Pro Pro Glu Trp Thr
   275 280 285
   Gly Gln Tyr Cys Thr Glu Asp Val Asp Glu Cys Gln Leu Met Pro Asn
   290 295 300
   Ala Cys Gln Asn Gly Gly Thr Cys His Asn Thr His Gly Gly Tyr Asn
   305 310 315 320
   Cys Val Cys Val Asn Gly Trp Thr Gly Glu Asp Cys Ser Glu Asn Ile
   325 330 335
   Asp Asp Cys Ala Ser Ala Ala Cys Phe His Gly Ala Thr Cys His Asp
   340 345 350
   Arg Val Ala Ser Phe Tyr Cys Glu Cys Pro His Gly Arg Thr Gly Leu
   355 360 365
   Leu Cys His Leu Asn Asp Ala Cys Ile Ser Asn Pro Cys Asn Glu Gly
   370 375 380
   Ser Asn Cys Asp Thr Asn Pro Val Asn Gly Lys Ala Ile Cys Thr Cys
   385 390 395 400
   Pro Ser Gly Tyr Thr Gly Pro Ala Cys Ser Gln Asp Val Asp Glu Cys
   405 410 415
   Ser Leu Gly Ala Asn Pro Cys Glu His Ala Gly Lys Cys Ile Asn Thr
   420 425 430
   Leu Gly Ser Phe Glu Cys Gln Cys Leu Gln Gly Tyr Thr Gly Pro Arg
           435                 440                 445
   Cys Glu Ile Asp Val Asn Glu Cys Val Ser Asn Pro Cys Gln Asn Asp
       450                 455                 460
   Ala Thr Cys Leu Asp Gln Ile Gly Glu Phe Gln Cys Ile Cys Met Pro
   465                 470                 475                 480
   Gly Tyr Glu Gly Val His Cys Glu Val Asn Thr Asp Glu Cys Ala Ser
                   485                 490                 495
   Ser Pro Cys Leu His Asn Gly Arg Cys Leu Asp Lys Ile Asn Glu Phe
               500                 505                 510
   Gln Cys Glu Cys Pro Thr Gly Phe Thr Gly His Leu Cys Gln Tyr Asp
           515                 520                 525
   Val Asp Glu Cys Ala Ser Thr Pro Cys Lys Asn Gly Ala Lys Cys Leu
       530                 535                 540
   Asp Gly Pro Asn Thr Tyr Thr Cys Val Cys Thr Glu Gly Tyr Thr Gly
   545                 550                 555                 560
   Thr His Cys Glu Val Asp Ile Asp Glu Cys Asp Pro Asp Pro Cys His
                   565                 570                 575
   Tyr Gly Ser Cys Lys Asp Gly Val Ala Thr Phe Thr Cys Leu Cys Arg
               580                 585                 590
   Pro Gly Tyr Thr Gly His His Cys Glu Thr Asn Ile Asn Glu Cys Ser
           595                 600                 605
   Ser Gln Pro Cys Arg His Gly Gly Thr Cys Gln Asp Arg Asp Asn Ala
       610                 615                 620
   Tyr Leu Cys Phe Cys Leu Lys Gly Thr Thr Gly Pro Asn Cys Glu Ile
   625                 630                 635                 640
   Asn Leu Asp Asp Cys Ala Ser Ser Pro Cys Asp Ser Gly Thr Cys Leu
                   645                 650                 655
   Asp Lys Ile Asp Gly Tyr Glu Cys Ala Cys Glu Pro Gly Tyr Thr Gly
               660                 665                 670
   Ser Met Cys Asn Ile Asn Ile Asp Glu Cys Ala Gly Asn Pro Cys His
           675                 680                 685
   Asn Gly Gly Thr Cys Glu Asp Gly Ile Asn Gly Phe Thr Cys Arg Cys
       690                 695                 700
   Pro Glu Gly Tyr His Asp Pro Thr Cys Leu Ser Glu Val Asn Glu Cys
   705                 710                 715                 720
   Asn Ser Asn Pro Cys Val His Gly Ala Cys Arg Asp Ser Leu Asn Gly
                   725                 730                 735
   Tyr Lys Cys Asp Cys Asp Pro Gly Trp Ser Gly Thr Asn Cys Asp Ile
               740                 745                 750
   Asn Asn Asn Glu Cys Glu Ser Asn Pro Cys Val Asn Gly Gly Thr Cys
           755                 760                 765
   Lys Asp Met Thr Ser Gly Tyr Val Cys Thr Cys Arg Glu Gly Phe Ser
       770                 775                 780
   Gly Pro Asn Cys Gln Thr Asn Ile Asn Glu Cys Ala Ser Asn Pro Cys
   785                 790                 795                 800
   Leu Asn Gln Gly Thr Cys Ile Asp Asp Val Ala Gly Tyr Lys Cys Asn
                   805                 810                 815
   Cys Leu Leu Pro Tyr Thr Gly Ala Thr Cys Glu Val Val Leu Ala Pro
               820                 825                 830
   Cys Ala Pro Ser Pro Cys Arg Asn Gly Gly Glu Cys Arg Gln Ser Glu
           835                 840                 845
   Asp Tyr Glu Ser Phe Ser Cys Val Cys Pro Thr Gly Trp Gln Ala Gly
       850                 855                 860
   Gln Thr Cys Glu Val Asp Ile Asn Glu Cys Val Leu Ser Pro Cys Arg
   865                 870                 875                 880
   His Gly Ala Ser Cys Gln Asn Thr His Gly Gly Tyr Arg Cys His Cys
                   885                 890                 895
   Gln Ala Gly Tyr Ser Gly Arg Asn Cys Glu Thr Asp Ile Asp Asp Cys
               900                 905                 910
   Arg Pro Asn Pro Cys His Asn Gly Gly Ser Cys Thr Asp Gly Ile Asn
           915                 920                 925
   Thr Ala Phe Cys Asp Cys Leu Pro Gly Phe Arg Gly Thr Phe Cys Glu
       930                 935                 940
   Glu Asp Ile Asn Glu Cys Ala Ser Asp Pro Cys Arg Asn Gly Ala Asn
   945                 950                 955                 960
   Cys Thr Asp Cys Val Asp Ser Tyr Thr Cys Thr Cys Pro Ala Gly Phe
                   965                 970                 975
   Ser Gly Ile His Cys Glu Asn Asn Thr Pro Asp Cys Thr Glu Ser Ser
               980                 985                 990
   Cys Phe Asn Gly Gly Thr Cys Val Asp Gly Ile Asn Ser Phe Thr Cys
           995                 1000                1005
   Leu Cys Pro Pro Gly Phe Thr Gly Ser Tyr Cys Gln His Asp Val Asn
       1010                1015                1020
   Glu Cys Asp Ser Gln Pro Cys Leu His Gly Gly Thr Cys Gln Asp Gly
   1025                1030                1035                1040
   Cys Gly Ser Tyr Arg Cys Thr Cys Pro Gln Gly Tyr Thr Gly Pro Asn
                   1045                1050                1055
   Cys Gln Asn Leu Val His Trp Cys Asp Ser Ser Pro Cys Lys Asn Gly
               1060                1065                1070
   Gly Lys Cys Trp Gln Thr His Thr Gln Tyr Arg Cys Glu Cys Pro Ser
           1075                1080                1085
   Gly Trp Thr Gly Leu Tyr Cys Asp Val Pro Ser Val Ser Cys Glu Val
       1090                1095                1100
   Ala Ala Gln Arg Gln Gly Val Asp Val Ala Arg Leu Cys Gln His Gly
   1105                1110                1115                1120
   Gly Leu Cys Val Asp Ala Gly Asn Thr His His Cys Arg Cys Gln Ala
                   1125                1130                1135
   Gly Tyr Thr Gly Ser Tyr Cys Glu Asp Leu Val Asp Glu Cys Ser Pro
               1140                1145                1150
   Ser Pro Cys Gln Asn Gly Ala Thr Cys Thr Asp Tyr Leu Gly Gly Tyr
           1155                1160                1165
   Ser Cys Lys Cys Val Ala Gly Tyr His Gly Val Asn Cys Ser Glu Glu
       1170                1175                1180
   Ile Asp Glu Cys Leu Ser His Pro Cys Gln Asn Gly Gly Thr Cys Leu
   1185                1190                1195                1200
   Asp Leu Pro Asn Thr Tyr Lys Cys Ser Cys Pro Arg Gly Thr Gln Gly
                   1205                1210                1215
   Val His Cys Glu Ile Asn Val Asp Asp Cys Asn Pro Pro Val Asp Pro
               1220                1225                1230
   Val Ser Arg Ser Pro Lys Cys Phe Asn Asn Gly Thr Cys Val Asp Gln
           1235                1240                1245
   Val Gly Gly Tyr Ser Cys Thr Cys Pro Pro Gly Phe Val Gly Glu Arg
       1250                1255                1260
   Cys Glu Gly Asp Val Asn Glu Cys Leu Ser Asn Pro Cys Asp Ala Arg
   1265                1270                1275                1280
   Gly Thr Gln Asn Cys Val Gln Arg Val Asn Asp Phe His Cys Glu Cys
                   1285                1290                1295
   Arg Ala Gly His Thr Gly Arg Arg Cys Glu Ser Val Ile Asn Gly Cys
               1300                1305                1310
   Lys Gly Lys Pro Cys Lys Asn Gly Gly Thr Cys Ala Val Ala Ser Asn
           1315                1320                1325
   Thr Ala Arg Gly Phe Ile Cys Lys Cys Pro Ala Gly Phe Glu Gly Ala
       1330                1335                1340
   Thr Cys Glu Asn Asp Ala Arg Thr Cys Gly Ser Leu Arg Cys Leu Asn
   1345                1350                1355                1360
   Gly Gly Thr Cys Ile Ser Gly Pro Arg Ser Pro Thr Cys Leu Cys Leu
                   1365                1370                1375
   Gly Pro Phe Thr Gly Pro Glu Cys Gln Phe Pro Ala Ser Ser Pro Cys
               1380                1385                1390
   Leu Gly Gly Asn Pro Cys Tyr Asn Gln Gly Thr Cys Glu Pro Thr Ser
           1395                1400                1405
   Glu Ser Pro Phe Tyr Arg Cys Leu Cys Pro Ala Lys Phe Asn Gly Leu
   1410 1415 1420
   Leu Cys His Ile Leu Asp Tyr Ser Phe Gly Gly Gly Ala Gly Arg Asp
   1425 1430 1435 1440
   Ile Pro Pro Pro Leu Ile Glu Glu Ala Cys Glu Leu Pro Glu Cys Gln
   1445 1450 1455
   Glu Asp Ala Gly Asn Lys Val Cys Ser Leu Gln Cys Asn Asn His Ala
   1460 1465 1470
   Cys Gly Trp Asp Gly Gly Asp Cys Ser Leu Asn Phe Asn Asp Pro Trp
   1475 1480 1485
   Lys Asn Cys Thr Gln Ser Leu Gln Cys Trp Lys Tyr Phe Ser Asp Gly
   1490 1495 1500
   His Cys Asp Ser Gln Cys Asn Ser Ala Gly Cys Leu Phe Asp Gly Phe
   1505 1510 1515 1520
   Asp Cys Gln Arg Ala Glu Gly Gln Cys Asn Pro Leu Tyr Asp Gln Tyr
   1525 1530 1535
   Cys Lys Asp His Phe Ser Asp Gly His Cys Asp Gln Gly Cys Asn Ser
   1540 1545 1550
   Ala Glu Cys Glu Trp Asp Gly Leu Asp Cys Ala Glu His Val Pro Glu
   1555 1560 1565
   Arg Leu Ala Ala Gly Thr Leu Val Val Val Val Leu Met Pro Pro Glu
   1570 1575 1580
   Gln Leu Arg Asn Ser Ser Phe His Phe Leu Arg Glu Leu Ser Arg Val
   1585 1590 1595 1600
   Leu His Thr Asn Val Val Phe Lys Arg Asp Ala His Gly Gln Gln Met
   1605 1610 1615
   Ile Phe Pro Tyr Tyr Gly Arg Glu Glu Glu Leu Arg Lys His Pro Ile
   1620 1625 1630
   Lys Arg Ala Ala Glu Gly Trp Ala Ala Pro Asp Ala Leu Leu Gly Gln
   1635 1640 1645
   Val Lys Ala Ser Leu Leu Pro Gly Gly Ser Glu Gly Gly Arg Arg Arg
   1650 1655 1660
   Arg Glu Leu Asp Pro Met Asp Val Arg Gly Ser Ile Val Tyr Leu Glu
   1665 1670 1675 1680
   Ile Asp Asn Arg Gln Cys Val Gln Ala Ser Ser Gln Cys Phe Gln Ser
   1685 1690 1695
   Ala Thr Asp Val Ala Ala Phe Leu Gly Ala Leu Ala Ser Leu Gly Ser
   1700 1705 1710
   Leu Asn Ile Pro Tyr Lys Ile Glu Ala Val Gln Ser Glu Thr Val Glu
   1715 1720 1725
   Pro Pro Pro Pro Ala Gln Leu His Phe Met Tyr Val Ala Ala Ala Ala
   1730 1735 1740
   Phe Val Leu Leu Phe Phe Val Gly Cys Gly Val Leu Leu Ser Arg Lys
   1745 1750 1755 1760
   Arg Arg Arg Gln His Gly Gln Leu Trp Phe Pro Glu Gly Phe Lys Val
   1765 1770 1775
   Ser Glu Ala Ser Lys Lys Lys Arg Arg Glu Pro Leu Gly Glu Asp Ser
   1780 1785 1790
   Val Gly Leu Lys Pro Leu Lys Asn Ala Ser Asp Gly Ala Leu Met Asp
   1795 1800 1805
   Asp Asn Gln Asn Glu Trp Gly Asp Glu Asp Leu Glu Thr Lys Lys Phe
   1810 1815 1820
   Arg Phe Glu Glu Pro Val Val Leu Pro Asp Leu Asp Asp Gln Thr Asp
   1825 1830 1835 1840
   His Arg Gln Trp Thr Gln Gln His Leu Asp Ala Ala Asp Leu Arg Met
   1845 1850 1855
   Ser Ala Met Ala Pro Thr Pro Pro Gln Gly Glu Val Asp Ala Asp Cys
   1860 1865 1870
   Met Asp Val Asn Val Arg Gly Pro Asp Gly Phe Thr Pro Leu Met Ile
   1875 1880 1885
   Ala Ser Cys Ser Gly Gly Gly Leu Glu Thr Gly Asn Ser Glu Glu Glu
   1890 1895 1900
   Glu Asp Ala Pro Ala Val Ile Ser Asp Phe Ile Tyr Gln Gly Ala Ser
   1905 1910 1915 1920
   Leu His Asn Gln Thr Asp Arg Thr Gly Glu Thr Ala Leu His Leu Ala
   1925 1930 1935
   Ala Arg Tyr Ser Arg Ser Asp Ala Ala Lys Arg Leu Leu Glu Ala Ser
   1940 1945 1950
   Ala Asp Ala Asn Ile Gln Asp Asn Met Gly Arg Thr Pro Leu His Ala
   1955 1960 1965
   Ala Val Ser Ala Asp Ala Gln Gly Val Phe Gln Ile Leu Ile Arg Asn
   1970 1975 1980
   Arg Ala Thr Asp Leu Asp Ala Arg Met His Asp Gly Thr Thr Pro Leu
   1985 1990 1995 2000
   Ile Leu Ala Ala Arg Leu Ala Val Glu Gly Met Leu Glu Asp Leu Ile
   2005 2010 2015
   Asn Ser His Ala Asp Val Asn Ala Val Asp Asp Leu Gly Lys Ser Ala
   2020 2025 2030
   Leu His Trp Ala Ala Ala Val Asn Asn Val Asp Ala Ala Val Val Leu
   2035 2040 2045
   Leu Lys Asn Gly Ala Asn Lys Asp Met Gln Asn Asn Arg Glu Glu Thr
   2050 2055 2060
   Pro Leu Phe Leu Ala Ala Arg Glu Gly Ser Tyr Glu Thr Ala Lys Val
   2065 2070 2075 2080
   Leu Leu Asp His Phe Ala Asn Arg Asp Ile Thr Asp His Met Asp Arg
   2085 2090 2095
   Leu Pro Arg Asp Ile Ala Gln Glu Arg Met His His Asp Ile Val Arg
   2100 2105 2110
   Leu Leu Asp Glu Tyr Asn Leu Val Arg Ser Pro Gln Leu His Gly Ala
   2115 2120 2125
   Pro Leu Gly Gly Thr Pro Thr Leu Ser Pro Pro Leu Cys Ser Pro Asn
   2130 2135 2140
   Gly Tyr Leu Gly Ser Leu Lys Pro Gly Val Gln Gly Lys Lys Val Arg
   2145 2150 2155 2160
   Lys Pro Ser Ser Lys Gly Leu Ala Cys Gly Ser Lys Glu Ala Lys Asp
   2165 2170 2175
   Leu Lys Ala Arg Arg Lys Lys Ser Gln Asp Gly Lys Gly Cys Leu Leu
   2180 2185 2190
   Asp Ser Ser Gly Met Leu Ser Pro Val Asp Ser Leu Glu Ser Pro His
   2195 2200 2205
   Gly Tyr Leu Ser Asp Val Ala Ser Pro Pro Leu Leu Pro Ser Pro Phe
   2210 2215 2220
   Gln Gln Ser Pro Ser Val Pro Leu Asn His Leu Pro Gly Met Pro Asp
   2225 2230 2235 2240
   Thr His Leu Gly Ile Gly His Leu Asn Val Ala Ala Lys Pro Glu Met
   2245 2250 2255
   Ala Ala Leu Gly Gly Gly Gly Arg Leu Ala Phe Glu Thr Gly Pro Pro
   2260 2265 2270
   Arg Leu Ser His Leu Pro Val Ala Ser Gly Thr Ser Thr Val Leu Gly
   2275 2280 2285
   Ser Ser Ser Gly Gly Ala Leu Asn Phe Thr Val Gly Gly Ser Thr Ser
   2290 2295 2300
   Leu Asn Gly Gln Cys Glu Trp Leu Ser Arg Leu Gln Ser Gly Met Val
   2305 2310 2315 2320
   Pro Asn Gln Tyr Asn Pro Leu Arg Gly Ser Val Ala Pro Gly Pro Leu
   2325 2330 2335
   Ser Thr Gln Ala Pro Ser Leu Gln His Gly Met Val Gly Pro Leu His
   2340 2345 2350
   Ser Ser Leu Ala Ala Ser Ala Leu Ser Gln Met Met Ser Tyr Gln Gly
   2355 2360 2365
   Leu Pro Ser Thr Arg Leu Ala Thr Gln Pro His Leu Val Gln Thr Gln
   2370 2375 2380
   Gln Val Gln Pro Gln Asn Leu Gln Met Gln Gln Gln Asn Leu Gln Pro
   2385                2390                2395                2400
   Ala Asn Ile Gln Gln Gln Gln Ser Leu Gln Pro Pro Pro Pro Pro Pro
                   2405                2410                2415
   Gln Pro His Leu Gly Val Ser Ser Ala Ala Ser Gly His Leu Gly Arg
               2420                2425                2430
   Ser Phe Leu Ser Gly Glu Pro Ser Gln Ala Asp Val Gln Pro Leu Gly
           2435                2440                2445
   Pro Ser Ser Leu Ala Val His Thr Ile Leu Pro Gln Glu Ser Pro Ala
       2450                2455                2460
   Leu Pro Thr Ser Leu Pro Ser Ser Leu Val Pro Pro Val Thr Ala Ala
   2465                2470                2475                2480
   Gln Phe Leu Thr Pro Pro Ser Gln His Ser Tyr Ser Ser Pro Val Asp
                   2485                2490                2495
   Asn Thr Pro Ser His Gln Leu Gln Val Pro Glu His Pro Phe Leu Thr
               2500                2505                2510
   Pro Ser Pro Glu Ser Pro Asp Gln Trp Ser Ser Ser Ser Pro His Ser
           2515                2520                2525
   Asn Val Ser Asp Trp Ser Glu Gly Val Ser Ser Pro Pro Thr Ser Met
       2530                2535                2540
   Gln Ser Gln Ile Ala Arg Ile Pro Glu Ala Phe Lys
   2545                2550                2555
<210> 7
   <211> 6009
   <212> DNA
   <213> Homo sapien
<400> 7
   atgcagcccc cttcactgct gctgctgctg ctgctgctgc tgctatgtgt ctcagtggtc 60
   agacccagag ggctgctgtg tgggagtttc ccagaaccct gtgccaatgg aggcacctgc 120
   ctgagcctgt ctctgggaca agggacctgc cagtgtgccc ctggcttcct gggtgagacg 180
   tgccagtttc ctgacccctg ccagaacgcc cagctctgcc aaaatggagg cagctgccaa 240
   gccctgcttc ccgctcccct agggctcccc agctctccct ctccattgac acccagcttc 300
   ttgtgcactt gcctccctgg cttcactggt gagagatgcc aggccaagct tgaagaccct 360
   tgtcctccct ccttctgttc caaaaggggc cgctgccaca tccaggcctc gggccgccca 420
   cagtgctcct gcatgcctgg atggacaggt gagcagtgcc agcttcggga cttctgttca 480
   gccaacccat gtgttaatgg aggggtgtgt ctggccacgt acccccagat ccagtgccac 540
   tgcccaccgg gcttcgaggg ccatgcctgt gaacgtgatg tcaacgagtg cttccaggac 600
   ccaggaccct gccccaaagg cacctcctgc cataacaccc tgggctcctt ccagtgcctc 660
   tgccctgtgg ggcaggaggg tccacgttgt gagctgcggg caggaccctg ccctcctagg 720
   ggctgttcga atgggggcac ctgccagctg atgccagaga aagactccac ctttcacctc 780
   tgcctctgtc cccaggtttt cataggcccg ggctgtgagg tgaatccaga caactgtgtc 840
   agccaccaat gtcagaatgg gggcacttgc caggatgggc tggacaccta cacctgcctc 900
   tgcccagaaa cctggacagg ctgggactgc tccgaagatg tggatgagtg tgaggcccag 960
   ggtccccctc actgcagaaa cgggggcacc tgccagaact ctgctggtag ctttcactgc 1020
   gtgtgtgtga gtggctgggg gggcacaagc tgtgaggaga acctggatga ctgtattgct 1080
   gccacctgtg ccccgggatc cacctgcatt gaccgggtgg gctctttctc ctgcctctgc 1140
   ccacctggac gcacaggact cctgtgccac ttggaagaca tgtgtctgag ccagccgtgc 1200
   catggggatg cccaatgcag caccaacccc ctcacaggct ccacactctg cctgtgtcag 1260
   cctggctatt cggggcccac ctgccaccag gacctggacg agtgtctgat ggcccagcaa 1320
   ggcccaagtc cctgtgaaca tggcggttcc tgcctcaaca ctcctggctc cttcaactgc 1380
   ctctgtccac ctggctacac aggctcccgt tgtgaggctg atcacaatga gtgcctctcc 1440
   cagccctgcc acccaggaag cacctgtctg gacctacttg ccaccttcca ctgcctctgc 1500
   ccgccaggct tagaagggca gctctgtgag gtggagacca acgagtgtgc ctcagctccc 1560
   tgcctgaacc acgcggattg ccatgacctg ctcaacggct tccagtgcat ctgcctgcct 1620
   ggattctccg gcacccgatg tgaggaggat atcgatgagt gcagaagctc tccctgtgcc 1680
   aatggtgggc agtgccagga ccagcctgga gccttccact gcaagtgtct cccaggcttt 1740
   gaagggccac gctgtcaaac agaggtggat gagtgcctga gtgacccatg tcccgttgga 1800
   gccagctgcc ttgatcttcc aggagccttc ttttgcctct gcccctctgg tttcacaggc 1860
   cagctctgtg aggttcccct gtgtgctccc aacctgtgcc agcccaagca gatatgtaag 1920
   gaccagaaag acaaggccaa ctgcctctgt cctgatggaa gccctggctg tgccccacct 1980
   gaggacaact gcacctgcca ccacgggcac tgccagagat cctcatgtgt gtgtgacgtg 2040
   ggttggacgg ggccagagtg tgaggcagag ctagggggct gcatctctgc accctgtgcc 2100
   catgggggga cctgctaccc ccagccctct ggctacaact gcacctgccc tacaggctac 2160
   acaggaccca cctgtagtga ggagatgaca gcttgtcact cagggccatg tctcaatggc 2220
   ggctcctgca accctagccc tggaggctac tactgcacct gccctccaag ccacacaggg 2280
   ccccagtgcc aaaccagcac tgactactgt gtgtctgccc cgtgcttcaa tgggggtacc 2340
   tgtgtgaaca ggcctggcac cttctcctgc ctctgtgcca tgggcttcca gggcccgcgc 2400
   tgtgagggaa agctccgccc cagctgtgca gacagcccct gtaggaatag ggcaacctgc 2460
   caggacagcc ctcagggtcc ccgctgcctc tgccccactg gctacaccgg aggcagctgc 2520
   cagactctga tggacttatg tgcccagaag ccctgcccac gcaattccca ctgcctccag 2580
   actgggccct ccttccactg cttgtgcctc cagggatgga ccgggcctct ctgcaacctt 2640
   ccactgtcct cctgccagaa ggctgcactg agccaaggca tagacgtctc ttccctttgc 2700
   cacaatggag gcctctgtgt cgacagcggc ccctcctatt tctgccactg cccccctgga 2760
   ttccaaggca gcctgtgcca ggatcacgtg aacccatgtg agtccaggcc ttgccagaac 2820
   ggggccacct gcatggccca gcccagtggg tatctctgcc agtgtgcccc aggctacgat 2880
   ggacagaact gctcaaagga actcgatgct tgtcagtccc aaccctgtca caaccatgga 2940
   acctgtactc ccaaacctgg aggcttccac tgtgcctgcc ctccaggctt tgtggggcta 3000
   cgctgtgagg gagacgtgga cgagtgtctg gaccagccct gccaccccac aggcactgca 3060
   gcctgccact ctctggccaa tgccttctac tgccagtgtc tgcctggaca cacaggccag 3120
   tggtgtgagg tggagataga cccctgccac agccaaccct gctttcatgg agggacctgt 3180
   gaggccacag caggatcacc cctgggtttc atctgccact gccccaaggg ttttgaaggc 3240
   cccacctgca gccacagggc cccttcctgc ggcttccatc actgccacca cggaggcctg 3300
   tgtctgccct cccctaagcc aggcttccca ccacgctgtg cctgcctcag tggctatggg 3360
   ggtcctgact gcctgacccc accagctcct aaaggctgtg gccctccctc cccatgccta 3420
   tacaatggca gctgctcaga gaccacgggc ttggggggcc caggctttcg atgctcctgc 3480
   cctcacagct ctccagggcc ccggtgtcag aaacccggag ccaaggggtg tgagggcaga 3540
   agtggagatg gggcctgcga tgctggctgc agtggcccgg gaggaaactg ggatggaggg 3600
   gactgctctc tgggagtccc agacccctgg aagggctgcc cctcccactc tcggtgctgg 3660
   cttctcttcc gggacggcca gtgccaccca cagtgtgact ctgaagagtg tctgtttgat 3720
   ggctacgact gtgagacccc tccagcctgc actccagcct atgaccagta ctgccatgat 3780
   cacttccaca acgggcactg tgagaaaggc tgcaacactg cagagtgtgg ctgggatgga 3840
   ggtgactgca ggcctgaaga tggggaccca gagtgggggc cctccctggc cctgctggtg 3900
   gtactgagcc ccccagccct agaccagcag ctgtttgccc tggcccgggt gctgtccctg 3960
   actctgaggg taggactctg ggtaaggaag gatcgtgatg gcagggacat ggtgtacccc 4020
   tatcctgggg cccgggctga agaaaagcta ggaggaactc gggaccccac ctatcaggag 4080
   agagcagccc ctcaaacaca gcccctgggc aaggagaccg actccctcag tgctgggttt 4140
   gtggtggtca tgggtgtgga tttgtcccgc tgtggccctg accacccggc atcccgctgt 4200
   ccctgggacc ctgggcttct actccgcttc cttgctgcga tggctgcagt gggagccctg 4260
   gagcccctgc tgcctggacc actgctggct gtccaccctc atgcagggac cgcaccccct 4320
   gccaaccagc ttccctggcc tgtgctgtgc tccccagtgg ccggggtgat tctcctggcc 4380
   ctaggggctc ttctcgtcct ccagctcatc cggcgtcgac gccgagagca tggagctctc 4440
   tggctgcccc ctggtttcac tcgacggcct cggactcagt cagctcccca ccgacgccgg 4500
   cccccactag gcgaggacag cattggtctc aaggcactga agccaaaggc agaagttgat 4560
   gaggatggag ttgtgatgtg ctcaggccct gaggagggag aggaggtggg ccaggctgaa 4620
   gaaacaggcc caccctccac gtgccagctc tggtctctga gtggtggctg tggggcgctc 4680
   cctcaggcag ccatgctaac tcctccccag gaatctgaga tggaagcccc tgacctggac 4740
   acccgtggac ctgatggggt gacacccctg atgtcagcag tttgctgtgg ggaagtacag 4800
   tccgggacct tccaaggggc atggttggga tgtcctgagc cctgggaacc tctgctggat 4860
   ggaggggcct gtccccaggc tcacaccgtg ggcactgggg agacccccct gcacctggct 4920
   gcccgattct cccggccaac cgctgcccgc cgcctccttg aggctggagc caaccccaac 4980
   cagccagacc gggcagggcg cacacccctt catgctgctg tggctgctga tgctcgggag 5040
   gtctgccagc ttctgctccg tagcagacaa actgcagtgg acgctcgcac agaggacggg 5100
   accacaccct tgatgctggc tgccaggctg gcggtggaag acctggttga agaactgatt 5160
   gcagcccaag cagacgtggg ggccagagat aaatggggga aaactgcgct gcactgggct 5220
   gctgccgtga acaacgcccg agccgcccgc tcgcttctcc aggccggagc cgataaagat 5280
   gcccaggaca acagggagca gacgccgcta ttcctggcgg cgcgggaagg agcggtggaa 5340
   gtagcccagc tactgctggg gctgggggca gcccgagagc tgcgggacca ggctgggcta 5400
   gcgccggcgg acgtcgctca ccaacgtaac cactgggatc tgctgacgct gctggaaggg 5460
   gctgggccac cagaggcccg tcacaaagcc acgccgggcc gcgaggctgg gcccttcccg 5520
   cgcgcacgga cggtgtcagt aagcgtgccc ccgcatgggg gcggggctct gccgcgctgc 5580
   cggacgctgt cagccggagc aggccctcgt gggggcggag cttgtctgca ggctcggact 5640
   tggtccgtag acttggctgc gcgggggggc ggggcctatt ctcattgccg gagcctctcg 5700
   ggagtaggag caggaggagg cccgacccct cgcggccgta ggttttctgc aggcatgcgc 5760
   gggcctcggc ccaaccctgc gataatgcga ggaagatacg gagtggctgc cgggcgcgga 5820
   ggcagggtct caacggatga ctggccctgt gattgggtgg ccctgggagc ttgcggttct 5880
   gcctccaaca ttccgatccc gcctccttgc cttactccgt ccccggagcg gggatcacct 5940
   caacttgact gtggtccccc agccctccaa gaaatgccca taaaccaagg aggagagggt 6000
   aaaaaatag 6009
<210> 8
   <211> 2003
   <212> PRT
   <213> Homo sapien
<400> 8
   Met Gln Pro Pro Ser Leu Leu Leu Leu Leu Leu Leu Leu Leu Leu Leu
   1 5 10 15
   Cys Val Ser Val Val Arg Pro Arg Gly Leu Leu Cys Gly Ser Phe Pro
   20 25 30
   Glu Pro Cys Ala Asn Gly Gly Thr Cys Leu Ser Leu Ser Leu Gly Gln
   35 40 45
   Gly Thr Cys Gln Cys Ala Pro Gly Phe Leu Gly Glu Thr Cys Gln Phe
   50 55 60
   Pro Asp Pro Cys Gln Asn Ala Gln Leu Cys Gln Asn Gly Gly Ser Cys
   65 70 75 80
   Gln Ala Leu Leu Pro Ala Pro Leu Gly Leu Pro Ser Ser Pro Ser Pro
   85 90 95
   Leu Thr Pro Ser Phe Leu Cys Thr Cys Leu Pro Gly Phe Thr Gly Glu
   100 105 110
   Arg Cys Gln Ala Lys Leu Glu Asp Pro Cys Pro Pro Ser Phe Cys Ser
   115 120 125
   Lys Arg Gly Arg Cys His Ile Gln Ala Ser Gly Arg Pro Gln Cys Ser
   130 135 140
   Cys Met Pro Gly Trp Thr Gly Glu Gln Cys Gln Leu Arg Asp Phe Cys
   145 150 155 160
   Ser Ala Asn Pro Cys Val Asn Gly Gly Val Cys Leu Ala Thr Tyr Pro
   165 170 175
   Gln Ile Gln Cys His Cys Pro Pro Gly Phe Glu Gly His Ala Cys Glu
   180 185 190
   Arg Asp Val Asn Glu Cys Phe Gln Asp Pro Gly Pro Cys Pro Lys Gly
   195 200 205
   Thr Ser Cys His Asn Thr Leu Gly Ser Phe Gln Cys Leu Cys Pro Val
   210 215 220
   Gly Gln Glu Gly Pro Arg Cys Glu Leu Arg Ala Gly Pro Cys Pro Pro
   225 230 235 240
   Arg Gly Cys Ser Asn Gly Gly Thr Cys Gln Leu Met Pro Glu Lys Asp
   245 250 255
   Ser Thr Phe His Leu Cys Leu Cys Pro Pro Gly Phe Ile Gly Pro Asp
   260 265 270
   Cys Glu Val Asn Pro Asp Asn Cys Val Ser His Gln Cys Gln Asn Gly
   275 280 285
   Gly Thr Cys Gln Asp Gly Leu Asp Thr Tyr Thr Cys Leu Cys Pro Glu
   290 295 300
   Thr Trp Thr Gly Trp Asp Cys Ser Glu Asp Val Asp Glu Cys Glu Thr
   305 310 315 320
   Gln Gly Pro Pro His Cys Arg Asn Gly Gly Thr Cys Gln Asn Ser Ala
   325 330 335
   Gly Ser Phe His Cys Val Cys Val Ser Gly Trp Gly Gly Thr Ser Cys
   340 345 350
   Glu Glu Asn Leu Asp Asp Cys Ile Ala Ala Thr Cys Ala Pro Gly Ser
           355                 360                 365
   Thr Cys Ile Asp Arg Val Gly Ser Phe Ser Cys Leu Cys Pro Pro Gly
       370                 375                 380
   Arg Thr Gly Leu Leu Cys His Leu Glu Asp Met Cys Leu Ser Gln Pro
   385                 390                 395                 400
   Cys His Gly Asp Ala Gln Cys Ser Thr Asn Pro Leu Thr Gly Ser Thr
                   405                 410                 415
   Leu Cys Leu Cys Gln Pro Gly Tyr Ser Gly Pro Thr Cys His Gln Asp
               420                 425                 430
   Leu Asp Glu Cys Leu Met Ala Gln Gln Gly Pro Ser Pro Cys Glu His
           435                 440                 445
   Gly Gly Ser Cys Leu Asn Thr Pro Gly Ser Phe Asn Cys Leu Cys Pro
       450                 455                 460
   Pro Gly Tyr Thr Gly Ser Arg Cys Glu Ala Asp His Asn Glu Cys Leu
   465                 470                 475                 480
   Ser Gln Pro Cys His Pro Gly Ser Thr Cys Leu Asp Leu Leu Ala Thr
                   485                 490                 495
   Phe His Cys Leu Cys Pro Pro Gly Leu Glu Gly Gln Leu Cys Glu Val
               500                 505                 510
   Glu Thr Asn Glu Cys Ala Ser Ala Pro Cys Leu Asn His Ala Asp Cys
           515                 520                 525
   His Asp Leu Leu Asn Gly Phe Gln Cys Ile Cys Leu Pro Gly Phe Ser
       530                 535                 540
   Gly Thr Arg Cys Glu Glu Asp Ile Asp Glu Cys Arg Ser Ser Pro Cys
   545                 550                 555                 560
   Ala Asn Gly Gly Gln Cys Gln Asp Gln Pro Gly Ala Phe His Cys Lys
                   565                 570                 575
   Cys Leu Pro Gly Phe Glu Gly Pro Arg Cys Gln Thr Glu Val Asp Glu
               580                 585                 590
   Cys Leu Ser Asp Pro Cys Pro Val Gly Ala Ser Cys Leu Asp Leu Pro
           595                 600                 605
   Gly Ala Phe Phe Cys Leu Cys Pro Ser Gly Phe Thr Gly Gln Leu Cys
       610                 615                 620
   Glu Val Pro Leu Cys Ala Pro Asn Leu Cys Gln Pro Lys Gln Ile Cys
   625                 630                 635                 640
   Lys Asp Gln Lys Asp Lys Ala Asn Cys Leu Cys Pro Asp Gly Ser Pro
                   645                 650                 655
   Gly Cys Ala Pro Pro Glu Asp Asn Cys Thr Cys His His Gly His Cys
               660                 665                 670
   Gln Arg Ser Ser Cys Val Cys Asp Val Gly Trp Thr Gly Pro Glu Cys
           675                 680                 685
   Glu Ala Glu Leu Gly Gly Cys Ile Ser Ala Pro Cys Ala His Gly Gly
       690                 695                 700
   Thr Cys Tyr Pro Gln Pro Ser Gly Tyr Asn Cys Thr Cys Pro Thr Gly
   705                 710                 715                 720
   Tyr Thr Gly Pro Thr Cys Ser Glu Glu Met Thr Ala Cys His Ser Gly
                   725                 730                 735
   Pro Cys Leu Asn Gly Gly Ser Cys Asn Pro Ser Pro Gly Gly Tyr Tyr
               740                 745                 750
   Cys Thr Cys Pro Pro Ser His Thr Gly Pro Gln Cys Gln Thr Ser Thr
           755                 760                 765
   Asp Tyr Cys Val Ser Ala Pro Cys Phe Asn Gly Gly Thr Cys Val Asn
       770                 775                 780
   Arg Pro Gly Thr Phe Ser Cys Leu Cys Ala Met Gly Phe Gln Gly Pro
   785                 790                 795                 800
   Arg Cys Glu Gly Lys Leu Arg Pro Ser Cys Ala Asp Ser Pro Cys Arg
                   805                 810                 815
   Asn Arg Ala Thr Cys Gln Asp Ser Pro Gln Gly Pro Arg Cys Leu Cys
               820                 825                 830
   Pro Thr Gly Tyr Thr Gly Gly Ser Cys Gln Thr Leu Met Asp Leu Cys
                   835                     840                     845
   Ala Gln Lys Pro Cys Pro Arg Asn Ser His Cys Leu Gln Thr Gly Pro
       850                     855                     860
   Ser Phe His Cys Leu Cys Leu Gln Gly Trp Thr Gly Pro Leu Cys Asn
   865                     870                     875                     880
   Leu Pro Leu Ser Ser Cys Gln Lys Ala Ala Leu Ser Gln Gly Ile Asp
                   885                     890                     895
   Val Ser Ser Leu Cys His Asn Gly Gly Leu Cys Val Asp Ser Gly Pro
               900                     905                     910
   Ser Tyr Phe Cys His Cys Pro Pro Gly Phe Gln Gly Ser Leu Cys Gln
           915                     920                     925
   Asp His Val Asn Pro Cys Glu Ser Arg Pro Cys Gln Asn Gly Ala Thr
       930                     935                     940
   Cys Met Ala Gln Pro Ser Gly Tyr Leu Cys Gln Cys Ala Pro Gly Tyr
   945                     950                     955                     960
   Asp Gly Gln Asn Cys Ser Lys Glu Leu Asp Ala Cys Gln Ser Gln Pro
                   965                     970                     975
   Cys His Asn His Gly Thr Cys Thr Pro Lys Pro Gly Gly Phe His Cys
               980                     985                     990
   Ala Cys Pro Pro Gly Phe Val Gly Leu Arg Cys Glu Gly Asp Val Asp
           995                     1000                    1005
   Glu Cys Leu Asp Gln Pro Cys His Pro Thr Gly Thr Ala Ala Cys His
       1010                    1015                    1020
   Ser Leu Ala Asn Ala Phe Tyr Cys Gln Cys Leu Pro Gly His Thr Gly
   1025                    1030                    1035                    1040
   Gln Trp Cys Glu Val Glu Ile Asp Pro Cys His Ser Gln Pro Cys Phe
                   1045                    1050                    1055
   His Gly Gly Thr Cys Glu Ala Thr Ala Gly Ser Pro Leu Gly Phe Ile
               1060                    1065                    1070
   Cys His Cys Pro Lys Gly Phe Glu Gly Pro Thr Cys Ser His Arg Ala
           1075                    1080                    1085
   Pro Ser Cys Gly Phe His His Cys His His Gly Gly Leu Cys Leu Pro
       1090                    1095                    1100
   Ser Pro Lys Pro Gly Phe Pro Pro Arg Cys Ala Cys Leu Ser Gly Tyr
   1105                    1110                    1115                    1120
   Gly Gly Pro Asp Cys Leu Thr Pro Pro Ala Pro Lys Gly Cys Gly Pro
                   1125                    1130                    1135
   Pro Ser Pro Cys Leu Tyr Asn Gly Ser Cys Ser Glu Thr Thr Gly Leu
               1140                    1145                    1150
   Gly Gly Pro Gly Phe Arg Cys Ser Cys Pro His Ser Ser Pro Gly Pro
           1155                    1160                    1165
   Arg Cys Gln Lys Pro Gly Ala Lys Gly Cys Glu Gly Arg Ser Gly Asp
       1170                    1175                    1180
   Gly Ala Cys Asp Ala Gly Cys Ser Gly Pro Gly Gly Asn Trp Asp Gly
   1185                    1190                    1195                    1200
   Gly Asp Cys Ser Leu Gly Val Pro Asp Pro Trp Lys Gly Cys Pro Ser
                   1205                    1210                    1215
   His Ser Arg Cys Trp Leu Leu Phe Arg Asp Gly Gln Cys His Pro Gln
               1220                    1225                    1230
   Cys Asp Ser Glu Glu Cys Leu Phe Asp Gly Tyr Asp Cys Glu Thr Pro
           1235                    1240                    1245
   Pro Ala Cys Thr Pro Ala Tyr Asp Gln Tyr Cys His Asp His Phe His
       1250                    1255                    1260
   Asn Gly His Cys Glu Lys Gly Cys Asn Thr Ala Glu Cys Gly Trp Asp
   1265                    1270                    1275                    1280
   Gly Gly Asp Cys Arg Pro Glu Asp Gly Asp Pro Glu Trp Gly Pro Ser
                   1285                    1290                    1295
   Leu Ala Leu Leu Val Val Leu Ser Pro Pro Ala Leu Asp Gln Gln Leu
               1300                    1305                    1310
   Phe Ala Leu Ala Arg Val Leu Ser Leu Thr Leu Arg Val Gly Leu Trp
           1315                    1320                    1325
   Val Arg Lys Asp Arg Asp Gly Arg Asp Met Val Tyr Pro Tyr Pro Gly
   1330 1335 1340
   Ala Arg Ala Glu Glu Lys Leu Gly Gly Thr Arg Asp Pro Thr Tyr Gln
   1345 1350 1355 1360
   Glu Arg Ala Ala Pro Gln Thr Gln Pro Leu Gly Lys Glu Thr Asp Ser
   1365 1370 1375
   Leu Ser Ala Gly Phe Val Val Val Met Gly Val Asp Leu Ser Arg Cys
   1380 1385 1390
   Gly Pro Asp His Pro Ala Ser Arg Cys Pro Trp Asp Pro Gly Leu Leu
   1395 1400 1405
   Leu Arg Phe Leu Ala Ala Met Ala Ala Val Gly Ala Leu Glu Pro Leu
   1410 1415 1420
   Leu Pro Gly Pro Leu Leu Ala Val His Pro His Ala Gly Thr Ala Pro
   1425 1430 1435 1440
   Pro Ala Asn Gln Leu Pro Trp Pro Val Leu Cys Ser Pro Val Ala Gly
   1445 1450 1455
   Val Ile Leu Leu Ala Leu Gly Ala Leu Leu Val Leu Gln Leu Ile Arg
   1460 1465 1470
   Arg Arg Arg Arg Glu His Gly Ala Leu Trp Leu Pro Pro Gly Phe Thr
   1475 1480 1485
   Arg Arg Pro Arg Thr Gln Ser Ala Pro His Arg Arg Arg Pro Pro Leu
   1490 1495 1500
   Gly Glu Asp Ser Ile Gly Leu Lys Ala Leu Lys Pro Lys Ala Glu Val
   1505 1510 1515 1520
   Asp Glu Asp Gly Val Val Met Cys Ser Gly Pro Glu Glu Gly Glu Glu
   1525 1530 1535
   Val Gly Gln Ala Glu Glu Thr Gly Pro Pro Ser Thr Cys Gln Leu Trp
   1540 1545 1550
   Ser Leu Ser Gly Gly Cys Gly Ala Leu Pro Gln Ala Ala Met Leu Thr
   1555 1560 1565
   Pro Pro Gln Glu Ser Glu Met Glu Ala Pro Asp Leu Asp Thr Arg Gly
   1570 1575 1580
   Pro Asp Gly Val Thr Pro Leu Met Ser Ala Val Cys Cys Gly Glu Val
   1585 1590 1595 1600
   Gln Ser Gly Thr Phe Gln Gly Ala Trp Leu Gly Cys Pro Glu Pro Trp
   1605 1610 1615
   Glu Pro Leu Leu Asp Gly Gly Ala Cys Pro Gln Ala His Thr Val Gly
   1620 1625 1630
   Thr Gly Glu Thr Pro Leu His Leu Ala Ala Arg Phe Ser Arg Pro Thr
   1635 1640 1645
   Ala Ala Arg Arg Leu Leu Glu Ala Gly Ala Asn Pro Asn Gln Pro Asp
   1650 1655 1660
   Arg Ala Gly Arg Thr Pro Leu His Ala Ala Val Ala Ala Asp Ala Arg
   1665 1670 1675 1680
   Glu Val Cys Gln Leu Leu Leu Arg Ser Arg Gln Thr Ala Val Asp Ala
   1685 1690 1695
   Arg Thr Glu Asp Gly Thr Thr Pro Leu Met Leu Ala Ala Arg Leu Ala
   1700 1705 1710
   Val Glu Asp Leu Val Glu Glu Leu Ile Ala Ala Gln Ala Asp Val Gly
   1715 1720 1725
   Ala Arg Asp Lys Trp Gly Lys Thr Ala Leu His Trp Ala Ala Ala Val
   1730 1735 1740
   Asn Asn Ala Arg Ala Ala Arg Ser Leu Leu Gln Ala Gly Ala Asp Lys
   1745 1750 1755 1760
   Asp Ala Gln Asp Asn Arg Glu Gln Thr Pro Leu Phe Leu Ala Ala Arg
   1765 1770 1775
   Glu Gly Ala Val Glu Val Ala Gln Leu Leu Leu Gly Leu Gly Ala Ala
   1780 1785 1790
   Arg Glu Leu Arg Asp Gln Ala Gly Leu Ala Pro Ala Asp Val Ala His
   1795 1800 1805
   Gln Arg Asn His Trp Asp Leu Leu Thr Leu Leu Glu Gly Ala Gly Pro
       1810                    1815                    1820
   Pro Glu Ala Arg His Lys Ala Thr Pro Gly Arg Glu Ala Gly Pro Phe
   1825                    1830                    1835                    1840
   Pro Arg Ala Arg Thr Val Ser Val Ser Val Pro Pro His Gly Gly Gly
                   1845                    1850                    1855
   Ala Leu Pro Arg Cys Arg Thr Leu Ser Ala Gly Ala Gly Pro Arg Gly
               1860                    1865                    1870
   Gly Gly Ala Cys Leu Gln Ala Arg Thr Trp Ser Val Asp Leu Ala Ala
           1875                    1880                    1885
   Arg Gly Gly Gly Ala Tyr Ser His Cys Arg Ser Leu Ser Gly Val Gly
       1890                    1895                    1900
   Ala Gly Gly Gly Pro Thr Pro Arg Gly Arg Arg Phe Ser Ala Gly Met
   1905                    1910                    1915                    1920
   Arg Gly Pro Arg Pro Asn Pro Ala Ile Met Arg Gly Arg Tyr Gly Val
                   1925                    1930                    1935
   Ala Ala Gly Arg Gly Gly Arg Val Ser Thr Asp Asp Trp Pro Cys Asp
               1940                    1945                    1950
   Trp Val Ala Leu Gly Ala Cys Gly Ser Ala Ser Asn Ile Pro Ile Pro
           1955                    1960                    1965
   Pro Pro Cys Leu Thr Pro Ser Pro Glu Arg Gly Ser Pro Gln Leu Asp
       1970                    1975                    1980
   Cys Gly Pro Pro Ala Leu Gln Glu Met Pro Ile Asn Gln Gly Gly Glu
   1985                    1990                    1995                    2000
   Gly Lys Lys
<210> 9
   <211> 19
   <212> DNA
   <213> Mus musculus
<400> 9
   gaggtccaag ccgaacctg 19
<210> 10
   <211> 21
   <212> DNA
   <213> mus musculus
<400> 10
   atcgctgatg tgcagttcac a 21
<210> 11
   <211> 20
   <212> DNA
   <213> Mus musculus
<400> 11
   cgctgccggc ctggattcac 20
<210> 12
   <211> 18
   <212> DNA
   <213> Mus musculus
<400> 12
   gatgcaagcc cggaagaa 18
<210> 13
   <211> 22
   <212> DNA
   <213> Mus musculus
<400> 13
   tcgcaattca gaaaggctac tg 22
<210> 14
   <211> 17
   <212> DNA
   <213> Mus musculus
<400> 14
   cgcagaggga tcataga 17
<210> 15
   <211> 19
   <212> DNA
   <213> Mus musculus
<400> 15
   ctacacatcg ccgctttcg 19
<210> 16
   <211> 18
   <212> DNA
   <213> Mus musculus
<400> 16
   cgcgtacttg gccttggt 18
<210> 17
   <211> 15
   <212> DNA
   <213> Mus musculus
<400> 17
   ccaccaggac atcgt 15
<210> 18
   <211> 530
   <212> PRT
   <213> Artificial Sequence
<220>
   <223> Synthetic
<400> 18
   Met Thr Pro Ala Ser Arg Ser Ala Cys Arg Trp Ala Leu Leu Leu Leu
   1               5                   10                  15
   Ala Val Leu Trp Pro Gln Gln Arg Ala Ala Gly Ser Gly Ile Phe Gln
               20                  25                  30
   Leu Arg Leu Gln Glu Phe Val Asn Gln Arg Gly Met Leu Ala Asn Gly
           35                  40                  45
   Gln Ser Cys Glu Pro Gly Cys Arg Thr Phe Phe Arg Ile Cys Leu Lys
       50                  55                  60
   His Phe Gln Ala Thr Phe Ser Glu Gly Pro Cys Thr Phe Gly Asn Val
   65                  70                  75                  80
   Ser Thr Pro Val Leu Gly Thr Asn Ser Phe Val Val Arg Asp Lys Asn
                       85                  90                  95
   Ser Gly Ser Gly Arg Asn Pro Leu Gln Leu Pro Phe Asn Phe Thr Trp
                   100                 105                 110
   Pro Gly Thr Phe Ser Leu Asn Ile Gln Ala Trp His Thr Pro Gly Asp
               115                 120                 125
   Asp Leu Arg Pro Glu Thr Ser Pro Gly Asn Ser Leu Ile Ser Gln Ile
       130                 135                 140
   Ile Ile Gln Gly Ser Leu Ala Val Gly Lys Ile Trp Arg Thr Asp Glu
   145                 150                 155                 160
   Gln Asn Asp Thr Leu Thr Arg Leu Ser Tyr Ser Tyr Arg Val Ile Cys
                   165                 170                 175
   Ser Asp Asn Tyr Tyr Gly Glu Ser Cys Ser Arg Leu Cys Lys Lys Arg
               180                 185                 190
   Asp Asp His Phe Gly His Tyr Glu Cys Gln Pro Asp Gly Ser Leu Ser
           195                 200                 205
   Cys Leu Pro Gly Trp Thr Gly Lys Tyr Cys Asp Gln Pro Ile Cys Leu
       210                 215                 220
   Ser Gly Cys His Glu Gln Asn Gly Tyr Cys Ser Lys Pro Asp Glu Cys
   225                 230                 235                 240
   Ile Cys Arg Pro Gly Trp Gln Gly Arg Leu Cys Asn Glu Cys Ile Pro
                   245                 250                 255
   His Asn Gly Cys Arg His Gly Thr Cys Ser Ile Pro Trp Gln Cys Ala
               260                 265                 270
   Cys Asp Glu Gly Trp Gly Gly Leu Phe Cys Asp Gln Asp Leu Asn Tyr
           275                 280                 285
   Cys Thr His His Ser Pro Cys Lys Asn Gly Ser Thr Cys Ser Asn Ser
       290                 295                 300
   Gly Pro Lys Gly Tyr Thr Cys Thr Cys Leu Pro Gly Tyr Thr Gly Glu
   305                 310                 315                 320
   His Cys Glu Leu Gly Leu Ser Lys Cys Ala Ser Asn Pro Cys Arg Asn
                   325                 330                 335
   Gly Gly Ser Cys Lys Asp Gln Glu Asn Ser Tyr His Cys Leu Cys Pro
               340                 345                 350
   Pro Gly Tyr Tyr Gly Gln His Cys Glu His Ser Thr Leu Thr Cys Ala
           355                 360                 365
   Asp Ser Pro Cys Phe Asn Gly Gly Ser Cys Arg Glu Arg Asn Gln Gly
       370                 375                 380
   Ser Ser Tyr Ala Cys Glu Cys Pro Pro Asn Phe Thr Gly Ser Asn Cys
   385                 390                 395                 400
   Glu Lys Lys Val Asp Arg Cys Thr Ser Asn Pro Cys Ala Asn Gly Gly
                   405                 410                 415
   Gln Cys Leu Asn Arg Gly Pro Ser Arg Thr Cys Arg Cys Arg Pro Gly
               420                 425                 430
   Phe Thr Gly Thr His Cys Glu Leu His Ile Ser Asp Cys Ala Arg Ser
           435                 440                 445
   Pro Cys Ala His Gly Gly Thr Cys His Asp Leu Glu Asn Gly Pro Val
       450                 455                 460
   Cys Thr Cys Pro Ala Gly Phe Ser Gly Arg Arg Cys Glu Val Arg Ile
   465                 470                 475                 480
   Thr His Asp Ala Cys Ala Ser Gly Pro Cys Phe Asn Gly Ala Thr Cys
                   485                 490                 495
   Tyr Thr Gly Leu Ser Pro Asn Asn Phe Val Cys Asn Cys Pro Tyr Gly
               500                 505                 510
   Phe Val Gly Ser Arg Cys Glu Phe Pro Val Gly Leu Pro Pro Ser Phe
           515                 520                 525
   Pro Trp
       530
<210> 19
   <211> 458
   <212> PRT
   <213> Artificial Sequence
<220>
   <223> Synthetic
<400> 19
   Met Val Ser Tyr Trp Asp Thr Gly Val Leu Leu Cys Ala Leu Leu Ser
   1 5 10 15
   Cys Leu Leu Leu Thr Gly Ser Ser Ser Gly Ser Asp Thr Gly Arg Pro
   20 25 30
   Phe Val Glu Met Tyr Ser Glu Ile Pro Glu Ile Ile His Met Thr Glu
   35 40 45
   Gly Arg Glu Leu Val Ile Pro Cys Arg Val Thr Ser Pro Asn Ile Thr
   50 55 60
   Val Thr Leu Lys Lys Phe Pro Leu Asp Thr Leu Ile Pro Asp Gly Lys
   65 70 75 80
   Arg Ile Ile Trp Asp Ser Arg Lys Gly Phe Ile Ile Ser Asn Ala Thr
   85 90 95
   Tyr Lys Glu Ile Gly Leu Leu Thr Cys Glu Ala Thr Val Asn Gly His
   100 105 110
   Leu Tyr Lys Thr Asn Tyr Leu Thr His Arg Gln Thr Asn Thr Ile Ile
   115 120 125
   Asp Val Val Leu Ser Pro Ser His Gly Ile Glu Leu Ser Val Gly Glu
   130 135 140
   Lys Leu Val Leu Asn Cys Thr Ala Arg Thr Glu Leu Asn Val Gly Ile
   145 150 155 160
   Asp Phe Asn Trp Glu Tyr Pro Ser Ser Lys His Gln His Lys Lys Leu
   165 170 175
   Val Asn Arg Asp Leu Lys Thr Gln Ser Gly Ser Glu Met Lys Lys Phe
   180 185 190
   Leu Ser Thr Leu Thr Ile Asp Gly Val Thr Arg Ser Asp Gln Gly Leu
   195 200 205
   Tyr Thr Cys Ala Ala Ser Ser Gly Leu Met Thr Lys Lys Asn Ser Thr
   210 215 220
   Phe Val Arg Val His Glu Lys Asp Lys Thr His Thr Cys Pro Pro Cys
   225 230 235 240
   Pro Ala Pro Glu Leu Leu Gly Gly Pro Ser Val Phe Leu Phe Pro Pro
   245 250 255
   Lys Pro Lys Asp Thr Leu Met Ile Ser Arg Thr Pro Glu Val Thr Cys
   260 265 270
   Val Val Val Asp Val Ser His Glu Asp Pro Glu Val Lys Phe Asn Trp
   275 280 285
   Tyr Val Asp Gly Val Glu Val His Asn Ala Lys Thr Lys Pro Arg Glu
   290 295 300
   Glu Gln Tyr Asn Ser Thr Tyr Arg Val Val Ser Val Leu Thr Val Leu
   305 310 315 320
   His Gln Asp Trp Leu Asn Gly Lys Glu Tyr Lys Cys Lys Val Ser Asn
   325 330 335
   Lys Ala Leu Pro Ala Pro Ile Glu Lys Thr Ile Ser Lys Ala Lys Gly
   340 345 350
   Gln Pro Arg Glu Pro Gln Val Tyr Thr Leu Pro Pro Ser Arg Asp Glu
   355 360 365
   Leu Thr Lys Asn Gln Val Ser Leu Thr Cys Leu Val Lys Gly Phe Tyr
   370 375 380
   Pro Ser Asp Ile Ala Val Glu Trp Glu Ser Asn Gly Gln Pro Glu Asn
   385 390 395 400
   Asn Tyr Lys Thr Thr Pro Pro Val Leu Asp Ser Asp Gly Ser Phe Phe
   405 410 415
   Leu Tyr Ser Lys Leu Thr Val Asp Lys Ser Arg Trp Gln Gln Gly Asn
   420 425 430
   Val Phe Ser Cys Ser Val Met His Glu Ala Leu His Asn His Tyr Thr
   435 440 445
   Gln Lys Ser Leu Ser Leu Ser Pro Gly Lys
   450 455
<210> 20
   <211> 227
   <212> PRT
   <213> Homo sapiens
<400> 20
   Asp Lys Thr His Thr Cys Pro Pro Cys Pro Ala Pro Glu Leu Leu Gly
   1 5 10 15
   Gly Pro Ser Val Phe Leu Phe Pro Pro Lys Pro Lys Asp Thr Leu Met
   20 25 30
   Ile Ser Arg Thr Pro Glu Val Thr Cys Val Val Val Asp Val Ser His
   35 40 45
   Glu Asp Pro Glu Val Lys Phe Asn Trp Tyr Val Asp Gly Val Glu Val
   50 55 60
   His Asn Ala Lys Thr Lys Pro Arg Glu Glu Gln Tyr Asn Ser Thr Tyr
   65 70 75 80
   Arg Val Val Ser Val Leu Thr Val Leu His Gln Asp Trp Leu Asn Gly
   85 90 95
   Lys Glu Tyr Lys Cys Lys Val Ser Asn Lys Ala Leu Pro Ala Pro Ile
   100 105 110
   Glu Lys Thr Ile Ser Lys Ala Lys Gly Gln Pro Arg Glu Pro Gln Val
   115 120 125
   Tyr Thr Leu Pro Pro Ser Arg Asp Glu Leu Thr Lys Asn Gln Val Ser
   130 135 140
   Leu Thr Cys Leu Val Lys Gly Phe Tyr Pro Ser Asp Ile Ala Val Glu
   145 150 155 160
   Trp Glu Ser Asn Gly Gln Pro Glu Asn Asn Tyr Lys Thr Thr Pro Pro
   165 170 175
   Val Leu Asp Ser Asp Gly Ser Phe Phe Leu Tyr Ser Lys Leu Thr Val
   180 185 190
   Asp Lys Ser Arg Trp Gln Gln Gly Asn Val Phe Ser Cys Ser Val Met
   195 200 205
   His Glu Ala Leu His Asn His Tyr Thr Gln Lys Ser Leu Ser Leu Ser
   210 215 220
   Pro Gly Lys
   225
<210> 21
   <211> 272
   <212> PRT
   <213> Artificial Sequence
<220>
   <223> Synthetic
<400> 21
   Val Ile Cys Ser Asp Asn Tyr Tyr Gly Asp Asn Cys Ser Arg Leu Cys
   1 5 10 15
   Lys Lys Arg Asn Asp His Phe Gly His Tyr Val Cys Gln Pro Asp Gly
   20 25 30
   Asn Leu Ser Cys Leu Pro Gly Trp Thr Gly Glu Tyr Cys Asp Lys Thr
   35 40 45
   His Thr Cys Pro Pro Cys Pro Ala Pro Glu Leu Leu Gly Gly Pro Ser
   50 55 60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Asp Glu Leu Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
<210> 22
   <211> 418
   <212> PRT
   <213> Artificial Sequence
<220>
   <223> Synthetic
<400> 22
   Ser Gly Val Phe Gln Leu Gln Leu Gln Glu Phe Ile Asn Glu Arg Gly
   1               5                   10                  15
   Val Leu Ala Ser Gly Arg Pro Cys Glu Pro Gly Cys Arg Thr Phe Phe
               20                  25                  30
   Arg Val Cys Leu Lys His Phe Gln Ala Val Val Ser Pro Gly Pro Cys
           35                  40                  45
   Thr Phe Gly Thr Val Ser Thr Pro Val Leu Gly Thr Asn Ser Phe Ala
       50                  55                  60
   Val Arg Asp Asp Ser Ser Gly Gly Gly Arg Asn Pro Leu Gln Leu Pro
   65                  70                  75                  80
   Phe Asn Phe Thr Trp Pro Gly Thr Phe Ser Leu Ile Ile Glu Ala Trp
                   85                  90                  95
   His Ala Pro Gly Asp Asp Leu Arg Pro Glu Ala Leu Pro Pro Asp Ala
               100                 105                 110
   Leu Ile Ser Lys Ile Ala Ile Gln Gly Ser Leu Ala Val Gly Gln Asn
           115                 120                 125
   Trp Leu Leu Asp Glu Gln Thr Ser Thr Leu Thr Arg Leu Arg Tyr Ser
       130                 135                 140
   Tyr Arg Val Ile Cys Ser Asp Asn Tyr Tyr Gly Asp Asn Cys Ser Arg
   145                 150                 155                 160
   Leu Cys Lys Lys Arg Asn Asp His Phe Gly His Tyr Val Cys Gln Pro
                   165                 170                 175
   Asp Gly Asn Leu Ser Cys Leu Pro Gly Trp Thr Gly Glu Tyr Cys Asp
               180                 185                 190
   Lys Thr His Thr Cys Pro Pro Cys Pro Ala Pro Glu Leu Leu Gly Gly
           195                     200                     205
   Pro Ser Val Phe Leu Phe Pro Pro Lys Pro Lys Asp Thr Leu Met Ile
       210                     215                     220
   Ser Arg Thr Pro Glu Val Thr Cys Val Val Val Asp Val Ser His Glu
   225                     230                     235                 240
   Asp Pro Glu Val Lys Phe Asn Trp Tyr Val Asp Gly Val Glu Val His
                   245                     250                     255
   Asn Ala Lys Thr Lys Pro Arg Glu Glu Gln Tyr Asn Ser Thr Tyr Arg
               260                     265                     270
   Val Val Ser Val Leu Thr Val Leu His Gln Asp Trp Leu Asn Gly Lys
           275                     280                     285
   Glu Tyr Lys Cys Lys Val Ser Asn Lys Ala Leu Pro Ala Pro Ile Glu
       290                     295                     300
   Lys Thr Ile Ser Lys Ala Lys Gly Gln Pro Arg Glu Pro Gln Val Tyr
   305                     310                     315                 320
   Thr Leu Pro Pro Ser Arg Asp Glu Leu Thr Lys Asn Gln Val Ser Leu
                   325                     330                     335
   Thr Cys Leu Val Lys Gly Phe Tyr Pro Ser Asp Ile Ala Val Glu Trp
               340                     345                     350
   Glu Ser Asn Gly Gln Pro Glu Asn Asn Tyr Lys Thr Thr Pro Pro Val
           355                     360                     365
   Leu Asp Ser Asp Gly Ser Phe Phe Leu Tyr Ser Lys Leu Thr Val Asp
       370                     375                     380
   Lys Ser Arg Trp Gln Gln Gly Asn Val Phe Ser Cys Ser Val Met His
   385                     390                     395                 400
   Glu Ala Leu His Asn His Tyr Thr Gln Lys Ser Leu Ser Leu Ser Pro
                   405                     410                     415
   Gly Lys
<210> 23
   <211> 410
   <212> PRT
   <213> Artificial Sequence
<220>
   <223> Synthetic
<400> 23
   Gln Gln Pro Ile Cys Leu Ser Gly Cys His Glu Gln Asn Gly Tyr Cys
   1               5                   10                  15
   Ser Lys Pro Ala Glu Cys Leu Cys Arg Pro Gly Trp Gln Gly Arg Leu
               20                  25                  30
   Cys Asn Glu Cys Ile Pro His Asn Gly Cys Arg His Gly Thr Cys Ser
           35                  40                  45
   Thr Pro Trp Gln Cys Thr Cys Asp Glu Gly Trp Gly Gly Leu Phe Cys
       50                  55                  60
   Asp Gln Asp Leu Asn Tyr Cys Thr His His Ser Pro Cys Lys Asn Gly
   65                  70                  75                  80
   Ala Thr Cys Ser Asn Ser Gly Gln Arg Ser Tyr Thr Cys Thr Cys Arg
                   85                  90                  95
   Pro Gly Tyr Thr Gly Val Asp Cys Glu Leu Glu Leu Ser Glu Cys Asp
               100                 105                 110
   Ser Asn Pro Cys Arg Asn Gly Gly Ser Cys Lys Asp Gln Glu Asp Gly
           115                 120                 125
   Tyr His Cys Leu Cys Pro Pro Gly Tyr Tyr Gly Leu His Cys Glu His
       130                 135                 140
   Ser Thr Leu Ser Cys Ala Asp Ser Pro Cys Phe Asn Gly Gly Ser Cys
   145                 150                 155                 160
   Arg Glu Arg Asn Gln Gly Ala Asn Tyr Ala Cys Glu Cys Pro Pro Asn
                           165                     170                     175
   Phe Thr Gly Ser Asn Cys Glu Asp Lys Thr His Thr Cys Pro Pro Cys
                   180                     185                     190
   Pro Ala Pro Glu Leu Leu Gly Gly Pro Ser Val Phe Leu Phe Pro Pro
               195                     200                     205
   Lys Pro Lys Asp Thr Leu Met Ile Ser Arg Thr Pro Glu Val Thr Cys
       210                     215                     220
   Val Val Val Asp Val Ser His Glu Asp Pro Glu Val Lys Phe Asn Trp
   225                     230                     235                     240
   Tyr Val Asp Gly Val Glu Val His Asn Ala Lys Thr Lys Pro Arg Glu
                   245                     250                     255
   Glu Gln Tyr Asn Ser Thr Tyr Arg Val Val Ser Val Leu Thr Val Leu
               260                     265                     270
   His Gln Asp Trp Leu Asn Gly Lys Glu Tyr Lys Cys Lys Val Ser Asn
           275                     280                     285
   Lys Ala Leu Pro Ala Pro Ile Glu Lys Thr Ile Ser Lys Ala Lys Gly
       290                     295                     300
   Gln Pro Arg Glu Pro Gln Val Tyr Thr Leu Pro Pro Ser Arg Asp Glu
   305                     310                     315                     320
   Leu Thr Lys Asn Gln Val Ser Leu Thr Cys Leu Val Lys Gly Phe Tyr
                   325                     330                     335
   Pro Ser Asp Ile Ala Val Glu Trp Glu Ser Asn Gly Gln Pro Glu Asn
               340                     345                     350
   Asn Tyr Lys Thr Thr Pro Pro Val Leu Asp Ser Asp Gly Ser Phe Phe
           355                     360                     365
   Leu Tyr Ser Lys Leu Thr Val Asp Lys Ser Arg Trp Gln Gln Gly Asn
       370                     375                     380
   Val Phe Ser Cys Ser Val Met His Glu Ala Leu His Asn His Tyr Thr
   385                     390                     395                     400
   Gln Lys Ser Leu Ser Leu Ser Pro Gly Lys
                   405                     410
<210> 24
   <211> 370
   <212> PRT
   <213> Artificial Sequence
<220>
   <223> Synthetic
<400> 24
   Gln Gln Pro Ile Cys Leu Ser Gly Cys His Glu Gln Asn Gly Tyr Cys
   1               5                   10                  15
   Ser Lys Pro Ala Glu Cys Leu Cys Arg Pro Gly Trp Gln Gly Arg Leu
               20                  25                  30
   Cys Asn Glu Cys Ile Pro His Asn Gly Cys Arg His Gly Thr Cys Ser
           35                  40                  45
   Thr Pro Trp Gln Cys Thr Cys Asp Glu Gly Trp Gly Gly Leu Phe Cys
       50                  55                  60
   Asp Gln Asp Leu Asn Tyr Cys Thr His His Ser Pro Cys Lys Asn Gly
   65                  70                  75                  80
   Ala Thr Cys Ser Asn Ser Gly Gln Arg Ser Tyr Thr Cys Thr Cys Arg
                   85                  90                  95
   Pro Gly Tyr Thr Gly Val Asp Cys Glu Leu Glu Leu Ser Glu Cys Asp
               100                 105                 110
   Ser Asn Pro Cys Arg Asn Gly Gly Ser Cys Lys Asp Gln Glu Asp Gly
           115                 120                 125
   Tyr His Cys Leu Cys Pro Pro Gly Tyr Tyr Gly Leu His Cys Glu Asp
       130                 135                 140
   Lys Thr His Thr Cys Pro Pro Cys Pro Ala Pro Glu Leu Leu Gly Gly
   145                     150                     155                     160
   Pro Ser Val Phe Leu Phe Pro Pro Lys Pro Lys Asp Thr Leu Met Ile
                   165                     170                     175
   Ser Arg Thr Pro Glu Val Thr Cys Val Val Val Asp Val Ser His Glu
               180                     185                     190
   Asp Pro Glu Val Lys Phe Asn Trp Tyr Val Asp Gly Val Glu Val His
           195                     200                     205
   Asn Ala Lys Thr Lys Pro Arg Glu Glu Gln Tyr Asn Ser Thr Tyr Arg
       210                     215                     220
   Val Val Ser Val Leu Thr Val Leu His Gln Asp Trp Leu Asn Gly Lys
   225                     230                     235                     240
   Glu Tyr Lys Cys Lys Val Ser Asn Lys Ala Leu Pro Ala Pro Ile Glu
                   245                     250                     255
   Lys Thr Ile Ser Lys Ala Lys Gly Gln Pro Arg Glu Pro Gln Val Tyr
               260                     265                     270
   Thr Leu Pro Pro Ser Arg Asp Glu Leu Thr Lys Asn Gln Val Ser Leu
           275                     280                     285
   Thr Cys Leu Val Lys Gly Phe Tyr Pro Ser Asp Ile Ala Val Glu Trp
       290                     295                     300
   Glu Ser Asn Gly Gln Pro Glu Asn Asn Tyr Lys Thr Thr Pro Pro Val
   305                     310                     315                     320
   Leu Asp Ser Asp Gly Ser Phe Phe Leu Tyr Ser Lys Leu Thr Val Asp
                   325                     330                     335
   Lys Ser Arg Trp Gln Gln Gly Asn Val Phe Ser Cys Ser Val Met His
               340                     345                     350
   Glu Ala Leu His Asn His Tyr Thr Gln Lys Ser Leu Ser Leu Ser Pro
           355                     360                     365
   Gly Lys
       370
<210> 25
   <211> 332
   <212> PRT
   <213> Artificial Sequence
<220>
   <223> synthetic
<400> 25
   Gln Gln Pro Ile Cys Leu Ser Gly Cys His Glu Gln Asn Gly Tyr Cys
   1               5                   10                  15
   Ser Lys Pro Ala Glu Cys Leu Cys Arg Pro Gly Trp Gln Gly Arg Leu
               20                  25                  30
   Cys Asn Glu Cys Ile Pro His Asn Gly Cys Arg His Gly Thr Cys Ser
           35                  40                  45
   Thr Pro Trp Gln Cys Thr Cys Asp Glu Gly Trp Gly Gly Leu Phe Cys
       50                  55                  60
   Asp Gln Asp Leu Asn Tyr Cys Thr His His Ser Pro Cys Lys Asn Gly
   65                  70                  75                  80
   Ala Thr Cys Ser Asn Ser Gly Gln Arg Ser Tyr Thr Cys Thr Cys Arg
                   85                  90                  95
   Pro Gly Tyr Thr Gly Val Asp Cys Glu Asp Lys Thr His Thr Cys Pro
               100                 105                 110
   Pro Cys Pro Ala Pro Glu Leu Leu Gly Gly Pro Ser Val Phe Leu Phe
           115                 120                 125
   Pro Pro Lys Pro Lys Asp Thr Leu Met Ile Ser Arg Thr Pro Glu Val
       130                 135                 140
   Thr Cys Val Val Val Asp Val Ser His Glu Asp Pro Glu Val Lys Phe
   145                 150                 155                 160
   Asn Trp Tyr Val Asp Gly Val Glu Val His Asn Ala Lys Thr Lys Pro
                       165                     170                     175
   Arg Glu Glu Gln Tyr Asn Ser Thr Tyr Arg Val Val Ser Val Leu Thr
                   180                 185                 190
   Val Leu His Gln Asp Trp Leu Asn Gly Lys Glu Tyr Lys Cys Lys Val
               195                 200                 205
   Ser Asn Lys Ala Leu Pro Ala Pro Ile Glu Lys Thr Ile Ser Lys Ala
       210                 215                 220
   Lys Gly Gln Pro Arg Glu Pro Gln Val Tyr Thr Leu Pro Pro Ser Arg
   225                 230                 235                 240
   Asp Glu Leu Thr Lys Asn Gln Val Ser Leu Thr Cys Leu Val Lys Gly
                   245                 250                 255
   Phe Tyr Pro Ser Asp Ile Ala Val Glu Trp Glu Ser Asn Gly Gln Pro
               260                 265                 270
   Glu Asn Asn Tyr Lys Thr Thr Pro Pro Val Leu Asp Ser Asp Gly Ser
           275                 280                 285
   Phe Phe Leu Tyr Ser Lys Leu Thr Val Asp Lys Ser Arg Trp Gln Gln
       290                 295                 300
   Gly Asn Val Phe Ser Cys Ser Val Met His Glu Ala Leu His Asn His
   305                 310                 315                 320
   Tyr Thr Gln Lys Ser Leu Ser Leu Ser Pro Gly Lys
                   325                 330
<210> 26
   <211> 292
   <212> PRT
   <213> Artificial Sequence
<220>
   <223> Synthetic
<400> 26
   Gln Gln Pro Ile Cys Leu Ser Gly Cys His Glu Gln Asn Gly Tyr Cys
   1               5                   10                  15
   Ser Lys Pro Ala Glu Cys Leu Cys Arg Pro Gly Trp Gln Gly Arg Leu
               20                  25                  30
   Cys Asn Glu Cys Ile Pro His Asn Gly Cys Arg His Gly Thr Cys Ser
           35                  40                  45
   Thr Pro Trp Gln Cys Thr Cys Asp Glu Gly Trp Gly Gly Leu Phe Cys
       50                  55                  60
   Asp Asp Lys Thr His Thr Cys Pro Pro Cys Pro Ala Pro Glu Leu Leu
   65                  70                  75                  80
   Gly Gly Pro Ser Val Phe Leu Phe Pro Pro Lys Pro Lys Asp Thr Leu
                   85                  90                  95
   Met Ile Ser Arg Thr Pro Glu Val Thr Cys Val Val Val Asp Val Ser
               100                 105                 110
   His Glu Asp Pro Glu Val Lys Phe Asn Trp Tyr Val Asp Gly Val Glu
           115                 120                 125
   Val His Asn Ala Lys Thr Lys Pro Arg Glu Glu Gln Tyr Asn Ser Thr
       130                 135                 140
   Tyr Arg Val Val Ser Val Leu Thr Val Leu His Gln Asp Trp Leu Asn
   145                 150                 155                 160
   Gly Lys Glu Tyr Lys Cys Lys Val Ser Asn Lys Ala Leu Pro Ala Pro
                   165                 170                 175
   Ile Glu Lys Thr Ile Ser Lys Ala Lys Gly Gln Pro Arg Glu Pro Gln
               180                 185                 190
   Val Tyr Thr Leu Pro Pro Ser Arg Asp Glu Leu Thr Lys Asn Gln Val
           195                 200                 205
   Ser Leu Thr Cys Leu Val Lys Gly Phe Tyr Pro Ser Asp Ile Ala Val
       210                 215                 220
   Glu Trp Glu Ser Asn Gly Gln Pro Glu Asn Asn Tyr Lys Thr Thr Pro
   225                 230                 235                 240
   Pro Val Leu Asp Ser Asp Gly Ser Phe Phe Leu Tyr Ser Lys Leu Thr
                   245                 250                 255
   Val Asp Lys Ser Arg Trp Gln Gln Gly Asn Val Phe Ser Cys Ser Val
               260                 265                 270
   Met His Glu Ala Leu His Asn His Tyr Thr Gln Lys Ser Leu Ser Leu
           275                 280                 285
   Ser Pro Gly Lys
       290

## Description

### BACKGROUND OF THE INVENTION

### Field of the Invention

This invention relates to inhibiting tumor growth with delta-like ligand 4 (Dll4) antagonists and VEGF inhibitors. Dll4 antagonists may be particularly useful for treating tumor growth in tumors which are unresponsive to other anti-tumor agents.

### Description of Related Art

The Notch-signaling pathway is a system for cell-to-cell communication used by a wide range of eukaryotes for many biological processes, such as differentiation, proliferation, and homeostasis. Delta like 4 (Dll4) or delta-like ligand 4 (Dll4) (hereinafter "Dll4") is a member of the Delta family of Notch ligands which exhibits highly selective expression by vascular endothelium (Shutter et al. (2000) Genes Develop. 14:1313-318). Dll4 is a ligand for Notch receptors, including Notch 1 and Notch 4. The nucleic acid and amino acid sequences for human and mouse Dll4 are shown in SEQ ID NO:1-2 and SEQ ID NO:3-4, respectively. Gene targeted Dll4 mice have been generated (Duarte et al. (2004) Genes & Dev. 18: doi: 10.11 01/gad.1239004; Krebs et al. (2004) Genes & Dev. 18: doi: 10.1101/gad.1239204; Gale et al. (2004) Proc Natl Acad Sci USA 101:15949-15954).
Patel et al. (Cancer Research, Vol. 65, pages 8690 - 8697 (2005)) describe upregulation of delta-like 4 ligand in human tumor vasculature and the role of basal expression in endothelial cell function. Gale et al. (PNAS, Vol. 101, pages 15949 - 15954 (2004)) describe that haplo insufficiency of delta-like 4 ligand results in embryonic lethality due to major defects in arterial and vascular development. Noguera et al. (Proceedings of the Annual Meeting of the American Association for Cancer Research, Vol. 46, SupplS., page 1104 (2005)) describe expression of delta-like 4 (Dll4) ligand in mouse tumor models. Mailhos et al (Differentiation, Vol. 69, pages 135 - 144 (2001)) describes Delta 4, as an endothelial specific Notch ligand expressed at sites of physiological and tumor angiogenesis. WO 03/050502 describes prospective identification and characterisation of breast cancer stem cells. WO 03/042246 describes an inhibitor of the notch signalling pathway in the manufacture of a medicament for use in the treatment of cancer.

### BRIEF SUMMARY OF THE INVENTION

The experiments described below show that Dll4 antagonists are effective in inhibiting tumor growth, particularly in tumors which are not responsive to other anti-tumor therapeutics, such as a vascular endothelial growth factor (VEGF) antagonist.

The invention features Dll4 antagonists capable of inhibiting Dll4.
The invention thus provides a delta-like ligand 4 (Dll4) antagonist and a vascular endothelial growth factor (VEGF) inhibitor for use in a method for treating tumor development or growth, wherein said Dll4 antagonist is a fusion protein comprising an extracellular domain of Dll4 and a human Fc domain of SEQ ID NO: 20, and wherein the VEGF inhibitor is a VEGF trap of SEQ ID NO: 19.

The extracellular domain of Dll4 is fused to a multimerizing component. The multimerizing component is a human Fc of SEQ ID NO:20. The fusion protein may optionally comprise a signal sequence, which may be native to the cell, recombinant, or synthetic.

The Dll4 antagonist is a modified Dll4 protein which is capable of binding its Notch receptor but such binding does not result in activation of the receptor.

The Dll4 antagonist of the invention may be particularly useful in treating tumors which are not responsive or are less than optimally responsive to other therapeutic agents. The Dll4 antagonist may block production of functional blood vessels and oxygen delivery to the tumors. The fusion protein of the invention comprises a fragment of the native extracellular region which retains the ability to bind to Notch receptors and lacks a transmembrane region and the cytoplasmic tail of Dll4. In one embodiment, the Dll4 antagonist of the invention is used therapeutically to treat tumors which are not responsive to treatment with a VEGF antagonist.

Other objects and advantages will become apparent from a review of the ensuing detailed description.

### BRIEF SUMMARY OF THE FIGURES

Fig. 1 shows that overexpression of Dll4-Fc by C6 tumor cells results in smaller C6 tumors.

Fig. 2 shows that systemically-delivered Dll4-Fc is highly effective in reducing HT1080 tumors relative to a receptor-based VEGF antagonist. Left hand panel: Dll4-Fc or VEGF Trap protein given at time of tumor implant, tumors harvested day 25;Right hand panel: Dll4-Fc or VEGF Trap protein given day 15 after implant, tumors harvested day 25.

Fig. 3 shows that purified Dll4-Fc protein or polyclonal Dll4 antibodies inhibits HT1080 tumor growth.

Fig. 4 shows inhibition of Dll4 binding to the Notch1 receptor by the polyclonal antibodies to Dll4, in a surface plasmon resonance (BiaCore®) assay.

### DETAILED DESCRIPTION

Before the present methods are described, it is to be understood that this invention is not limited to particular methods, and experimental conditions described, as such methods and conditions may vary. It is also to be understood that the terminology used herein is for the purpose of describing particular embodiments only, and is not intended to be limiting, since the scope of the present invention will be limited only by the appended claims.

As used in this specification and the appended claims, the singular forms "a", "an", and "the" include plural references unless the context clearly dictates otherwise. Thus for example, references to "a method" includes one or more methods, and/or steps of the type described herein and/or which will become apparent to those persons skilled in the art upon reading this disclosure and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described

### Definitions

By the term "Dll4-associated" or "Dll4-mediated" condition or disease is meant a condition which is affected directly or indirectly by modulation of Dll4 activity. More specifically, Dll4 is now shown to be involved in blood vessel growth and development. Accordingly, in one embodiment, a Dll4-associated condition treatable by the method of the invention is one in which it is desirable to inhibit or reduce Dll4-mediated blood vessel growth or development or maturation, e.g., to inhibit tumor development.

By the term "inhibitor" or "antagonist" is meant a substance which retards or prevents a chemical or physiological reaction or response. Inhibition of Dll4 activity may be direct, through inhibition of receptor activation with a blocking antibody, for example, or indirect, resulting from interference with expression of the gene encoding Dll4. Common inhibitors include but are not limited to antisense molecules, antibodies, soluble receptors, antagonists and their derivatives, and modified Dll4 ligands which bind their Notch receptor but are unable to activate signaling through such binding.

A "neutralizing" or "blocking" antibody, is intended to refer to an antibody whose binding to Dll4 results in inhibition of the biological activity of Dll4. This inhibition of the biological activity of Dll4 can be assessed by measuring one or more indicators of Dll4 biological activity. These indicators of Dll4 biological activity can be assessed by one or more of several standard *in vitro* or *in vivo* assays known in the art (see examples below). Preferably, the ability of an antibody to neutralize Dll4 activity is assessed by inhibition of Dll4 binding to a Notch receptor, such as Notch1.

### General Description

The Delta-like/Notch signaling pathway is necessary to establish an organized and hierarchical vasculature during development Targeted deletions of various Delta-like/Notch genes, including Dll4, result in mice that die during embryonic development due to severe vascular defects. Using microarray analysis, we found that Delta-like ligand 4 (Dll4) a VEGF-regulated gene in mouse xenograft tumor models. In addition, it was found that in these tumor models, Dll4 expression was significantly higher in tumor vessels compared to those in adjacent normal skin. To explore the effects of blocking Dll4/Notch signaling in tumors, xenograft studies were performed in mice, where a soluble Dll4-Fc molecule was delivered locally by retrovirally mediated over-expression in tumor cells or was delivered systemically using an adenoviral approach or by injecting purified protein. All methods of delivering Dll4-Fc resulted in reduced tumor growth compared to controls. Additionally, Dll4-Fc treated tumor vessels were more highly branched than controls, forming fine networks with dense vascular sprouting, but these vessels were less efficient than in those of control tumors. As revealed by array and Taqman™ analysis, these effects were associated with a decrease in Notch signaling. Similar effects on tumor growth were also observed using a polyclonal antibody solution that was injected into mice systemically. This polyclonal antibody solution was also found to inhibit binding of Dll4 to Notch1 receptor. Additionally, it was found that Dll4-Fc is more effective at reducing the growth of certain tumors than a receptor-based blocker of VEGF ("VEGF trap", US Patent No. 7,070,959). These findings show that Dll4 plays a key role in tumor growth, and support Dll4 as a target for the development of anti-angiogenic therapies.

### Dll4 Antagonists

Dll4 antagonists include antibodies to Dll4 and fragments thereof capable of blocking the binding of Dll4 to a Notch receptor (such as Notch1), fusion proteins comprising the extracellular domain of Dll4 fused to a multimerizing component, or fragments thereof, and peptides and peptibodies (see for example, US patent publication 2003/0229023 Oliner et al).

Dll4 antibodies. The term "immunoglobulin or antibody" as used herein refers to a mammalian, including human, polypeptide comprising a framework region from an immunoglobulin gene or fragments thereof that specifically binds and recognizes an antigen, which is a Dll4 protein or portion thereof. If the intended antibody or antibody-like protein will be used as a mammalian therapeutic, immunoglobulin binding regions should be derived from the corresponding mammalian immunoglobulins. If the molecule is intended for non-therapeutic use, such as for diagnostics and ELISAs, the immunoglobulin binding regions may be derived from either human or non-human mammals, such as mice. The human immunoglobulin genes or gene fragments include the kappa, lambda, alpha, gamma, delta, epsilon, and mu constant regions, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. Within each IgG class, there are different isotypes (eg. IgG₁, IgG₂, IgG₃, IgG₄) as well as allotypes thereof.

An exemplary immunoglobulin (antibody) structural unit of human IgG, comprises a tetramer. Each tetramer is composed of two identical pairs of polypeptide chains, each pair having one light chain (about 25 kD) and one heavy chain (about 50-70 kD). The N-terminus of each chain defines a variable region of about 100-110 or more amino acids primarily responsible for antigen recognition. The terms "variable light chain" (V_{L}) and variable heavy chain (V_{H}) refer to these light and heavy chains respectively.

Antibodies exist as intact immunoglobulins, or as a number of well-characterized fragments produced by digestion with various peptidases. For example, pepsin digests an antibody below the disulfide linkages in the hinge region to produce F(ab)'₂, a dimer of Fab which itself is a light chain joined to V_{H}-C_{H} by a disulfide bond. The F(ab)'₂ may be reduced under mild conditions to break the disulfide linkage in the hinge region, thereby converting the F(ab)'₂ dimer into an Fab' monomer. The Fab' monomer is essentially Fab with part of the hinge region. While various antibody fragments are defined in terms of the digestion of an intact antibody, one of skill will appreciate that such fragments may be synthesized de *novo* either chemically or by using recombinant DNA methodology. Thus, the terms antibody, as used herein, also includes antibody fragments either produced by the modification of whole antibodies, or those synthesized *de novo* using recombinant DNA methodologies (e.g., single chain Fv (scFv) single variable domains (Dabs)) or those identified using display libraries such as phage, *E. coli* or yeast display libraries (see, for example. McCafferty et al. (1990) Nature 348:552-554).

Methods for preparing antibodies are known to the art. See, for example, Kohler & Milstein (1975) Nature 256:495-497; Harlow & Lane (1988) Antibodies: a Laboratory Manual, Cold Spring Harbor Lab., Cold Spring Harbor, NY). Antibodies that are isolated from organisms other than humans, such as mice, rats, rabbits, cows, can be made more human-like through chimerization or humanization.

"Humanized" or chimeric forms of non-human (e.g., murine) antibodies are immunoglobulins, immunoglobulin chains or fragments thereof (such as Fv, Fab, Fab', F(ab')₂ or other antigen-binding subsequences of antibodies) that contain minimal sequences required for antigen binding derived from non-human immunoglobulin. They have the same or similar binding specificity and affinity as a mouse or other nonhuman antibody that provides the starting material for construction of a chimeric or humanized antibody. Chimeric antibodies are antibodies whose light and heavy chain genes have been constructed, typically by genetic engineering, from immunoglobulin gene segments belonging to different species. For example, the variable (V) segments of the genes from a mouse monoclonal antibody may be joined to human constant (C) segments, such as IgG1 and IgG4. A typical chimeric antibody is thus a hybrid protein consisting of the V or antigen-binding domain from a mouse antibody and the C or effector domain from a human antibody. Humanized antibodies have variable region framework residues substantially from a human antibody (termed an acceptor antibody) and complementarity determining regions (CDR regions) substantially from a mouse antibody, (referred to as the donor immunoglobulin). See, Queen et al., Proc. Natl. Acad Sci. USA 86:10029-10033 (1989) and WO 90/07861, U.S. patents 5,693,762, 5,693,761, 5,585,089, 5,530,101 and 5,225,539. The constant region(s), if present, are also substantially or entirely from a human immunoglobulin. The human variable domains are usually chosen from human antibodies whose framework sequences exhibit a high degree of sequence identity with the murine variable region domains from which the CDRs were derived. The heavy and light chain variable region framework residues can be derived from the same or different human antibody sequences. The human antibody sequences can be the sequences of naturally occurring human antibodies or can be consensus sequences of several human antibodies. See WO 92/22653. Certain amino acids from the human variable region framework residues are selected for substitution based on their possible influence on CDR conformation and/or binding to antigen. Investigation of such possible influences is by modeling, examination of the characteristics of the amino acids at particular locations, or empirical observation of the effects of substitution or mutagenesis of particular amino acids. For example, when an amino acid differs between a murine variable region framework residue and a selected human variable region framework residue, the human framework amino acid should usually be substituted by the equivalent framework amino acid from the mouse antibody when it is reasonably expected that the amino acid: (1) noncovalently binds antigen directly; (2) is adjacent to a CDR region; (3) otherwise interacts with a CDR region (e.g. is within about 6 Aof a CDR region), or (4) participates in the V_{L}-V_{H} interface. Other candidates for substitution are acceptor human framework amino acids that are unusual for a human immunoglobulin at that position. These amino acids can be substituted with amino acids from the equivalent position of the mouse donor antibody or from the equivalent positions of more typical human immunoglobulins. Other candidates for substitution are acceptor human framework amino acids that are unusual for a human immunoglobulin at that position. The variable region frameworks of humanized immunoglobulins usually show at least 85% sequence identity to a human variable region framework sequence or consensus of such sequences.

Methods for generating human antibodies include, for example, VelocImmune™ (Regeneron Pharmaceuticals), XenoMouse™ technology (Abgenix), the "minilocus" approach, and phage display. The VelocImmune™ technology (US 6,596,541) encompasses a method of generating a high specificity fully human antibody to a selected antigen. This technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody. In specific embodiment, the cell is a CHO cell.

The XenoMouse™ technology (Green et al. (1994) Nature Genetics 7:13-21) generates a mouse having both human variable and constant regions from both the heavy chain and kappa light chain loci. In an alternative approach, others have utilized a 'minilocus" approach in which an exogenous Ig locus is mimicked through inclusion of individual genes from the Ig locus (see, for example, US 5,545,807). The DNA encoding the variable regions can be isolated with or without being operably linked to the DNA encoding the human heavy and light chain constant region.

Alternatively, phage display or related display technologies can be used to identify antibodies, antibody fragments, such as variable domains, and heteromeric Fab fragments that specifically bind to Dll4. (see for example US patent publication 2003/0229023).

Screening and selection of preferred immunoglobulins (antibodies) can be conducted by a variety of methods known to the art. Initial screening for the presence of monoclonal antibodies specific to Dll4 may be conducted through the use of ELISA-based methods or phage display, for example. A secondary screen is preferably conducted to identify and select a desired monoclonal antibody. Secondary screening may be conducted with any suitable method known to the art. One preferred method, termed "Biosensor Modification-Assisted Profiling" ("BiaMAP") is described in U.S. patent application publication 2004/101920. BiaMAP allows rapid identification of hybridoma clones producing monoclonal antibodies with desired characteristics. More specifically, monoclonal antibodies are sorted into distinct epitope-related groups based on evaluation of antibody:antigen interactions. Alternatively, ELISA-based, bead-based, or Biacore®-based competition assays can be used to identify binding pairs that bind different epitopes of Dll4 and thus are likely to cooperate to bind the ligand with high affinity.

Dll4 fusion proteins. In the Dll4 antagonist fusion protein according to the invention, the multimerizing component is a human Fc of SEQ ID NO:20. The fusion protein may optionally comprise a signal sequence, which may comprise any sequence known to a skilled artisan for directing secretion of a polypeptide or protein from a cell, include natural or synthetic sequences. Generally, a signal sequence is placed at the beginning or amino-terminus of the fusion protein of the invention. Such a signal sequence may be native to the cell, recombinant, or synthetic. The components of the fusion protein of the invention may be connected directly to each other or connected via one or more spacer sequences. In one preferred embodiment, the components are fused directly to each other. In another preferred embodiment, the components are connected with a nucleic acid sequence encoding a spacer of 1-200 amino acids. Any spacer known to the art may be used to connect the protein components. A spacer sequence may also include a sequence used to enhance expression of the fusion protein, provide restriction sites, and allow component domains to form optimal tertiary and quaternary structures and/or to enhance the interaction of a component with its receptor. In one embodiment, the fusion protein comprises one or more peptide sequences between one or more components that is (are) between 1-25 amino acids.

The extracellular domain of Dll4 is composed of a Delta/Serrate/Lag-2 (DSL) domain and a tandem of eight epidermal growth factor (EGF)-like repeats. Generally, the EGF domains are recognized as occurring at about position 218-251 (domain 1), 252-282 (domain 2), 284-322 (domain 3), 324-360 (domain 4), and 362-400 (domain 5), with the DSL domain at about position 173-217 and the N-terminal domain at about position 27-172 of hDll4 (SEQ ID NO:2). In specific embodiments, the h Dll4 antagonist capable of inhibiting Dll4 activity is DSL-hFc comprising about amino acid 27 to about 172 of SEQ ID NO:2 fused to hFc (SEQ ID NO:20) (SEQ ID NO:21), N-terminal domain-DSL-hFc comprising about 27-217 of SEQ ID NO:2 fused to hFc (SEQ ID NO:22), EGF domains 1-5-hFc comprising about 218-400 fused to hFc (SEQ ID NO:23), EGF domains 1-4hFc comprising about 218-360 fused to hFc (SEQ ID NO:24), EGF domains 1-3-hFc comprising about 218-322 fused to hFc (SEQ ID NO:25), EGF domains 1-2-hFc comprising about 218-282 fused to hFc (SEQ ID NO:26), or variants thereof optionally comprising linkers between the domain components. The components of the fusion protein may also be arranged in a variety of configurations while retaining the ability to act as Dll4 antagonists.

### Methods of Administration

Described herein are methods of treatment comprising administering to a subject an effective amount of an agent of the present disclosure. In a preferred aspect, the agent is substantially purified (e.g., substantially free from substances that limit its effect or produce undesired side-effects). The subject is preferably an animal, e.g., such as cows, pigs, horses, chickens, cats, dogs, etc., and is preferably a mammal, and most preferably human.

Various delivery systems are known and can be used to administer an agent of the present disclosure, e.g., encapsulation in liposomes, microparticles, microcapsules, recombinant cells capable of expressing the compound, receptor-mediated endocytosis (see, e.g., Wu and Wu, 1987, J. Biol. Chem. 262:4429.4432), construction of a nucleic acid as part of a retroviral or other vector, etc. Methods of introduction can be enteral or parenteral and include but are not limited to intradermal, intramuscular, intraperitoneal, intravenous, subcutaneous, intranasal, epidural, and oral routes. The compounds may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce pharmaceutical compositions described herein into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compositions described herein locally to the area in need of treatment; this may be achieved, for example, and not by way of limitation, by local infusion during surgery, topical application, e.g., by injection, by means of a catheter, or by means of an implant, said implant being of a porous, nonporous, or gelatinous material, including membranes, such as sialastic membranes, fibers, or commercial skin substitutes.

In another embodiment, the active agent can be delivered in a vesicle, in particular a liposome (see Langer (1990) Science 249:1527-1533). In yet another embodiment, the active agent can be delivered in a controlled release system. In one embodiment, a pump may be used (see Langer (1990) supra). In another embodiment, polymeric materials can be used (see Howard et al. (1989) J. Neurosurg. 71:105). In another embodiment where the active agent of the present disclosure is a nucleic acid encoding a protein, the nucleic acid can be administered *in vivo* to promote expression of its encoded protein, by constructing it as part of an appropriate nucleic acid expression vector and administering it so that it becomes intracellular, e.g., by use of a retroviral vector (see, for example, U.S. Patent No. 4,980,286), or by direct injection, or by use of microparticle bombardment (e.g., a gene gun; Biolistic, Dupont), or coating with lipids or cell-surface receptors or transfecting agents, or by administering it in linkage to a homeobox-like peptide which is known to enter the nucleus (see e.g., Joliot et al., 1991, Proc. Natl. Acad. Sci. USA 88:1864-1868), etc. Alternatively, a nucleic acid can be introduced intracellularly and incorporated within host cell DNA for expression, by homologous recombination.

### Pharmaceutical Compositions

Described herein are pharmaceutical compositions. Such compositions comprise a therapeutically effective amount of an active agent, and a pharmaceutically acceptable carrier. The term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin.

In a preferred embodiment, the composition is formulated in accordance with routine procedures as a pharmaceutical composition adapted for intravenous administration to human beings. Where necessary, the composition may also include a solubilizing agent and a local anesthetic such as lidocaine to ease pain at the site of the injection. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The active agents of the present disclosure can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.

The amount of the active agent of the present disclosure which will be effective in the treatment of a Dll4-mediated condition can be determined by standard clinical techniques based on the present description. In addition, *in vitro* assays may optionally be employed to help identify optimal dosage ranges. The precise dose to be employed in the formulation will also depend on the route of administration, and the seriousness of the condition, and should be decided according to the judgment of the practitioner and each subject's circumstances. However, suitable dosage ranges for intravenous administration are generally about 0.5 to 20 milligrams of active compound per kilogram body weight. Suitable dosage ranges for intranasal administration are generally about 0.01 pg/kg body weight to 1 mg/kg body weight. Effective doses may be extrapolated from dose-response curves derived from *in vitro* or animal model test systems.

### Combination Therapies

As defined above, the present invention provides a Dll4 antagonist and a VEGF inhibitor for use in a method for treating tumor development or growth. The Dll4 antagonist and VEGF inhibitor for use according to the present invention may be administered in combination with one or more additional compounds or therapies. For example, multiple fusion proteins or anti- Dll4 antibodies can be co-administered, or be administered in conjunction with one or more therapeutic compounds. The VEGF antagonist is a VEGF trap of SEQ ID NO: 19. A VEGF trap (as described in WO 00/75319) is VEGFR1R2FcΔC1(a).

The term "cytotoxic agent" as used herein refers to a substance that inhibits or prevents the function of cells and/or causes destruction of cells. The term is intended to include radioactive isotopes (e.g. I¹³¹ I¹²⁵, Y⁹⁰ and Re¹⁸⁶), chemotherapeutic agents, and toxins such as enzymatically active toxins of bacterial, fungal, plant or animal origin, or fragments thereof.

Combination therapy includes administration of a single pharmaceutical dosage formulation which contains the Dll4 antagonist and the VEGF antagonist; as well as administration of a Dll4 antagonist and one or more additional agent(s) in its own separate pharmaceutical dosage formulation. For example, a Dll4 antagonist and a cytotoxic agent, a chemotherapeutic agent or a growth inhibitory agent can be administered to the patient together in a single dosage composition such as a combined formulation, or each agent can be administered in a separate dosage formulation. Where separate dosage formulations are used, a fusion protein for use according to the invention and one or more additional agents can be administered concurrently, or at separately staggered times, i.e., sequentially.

A "chemotherapeutic agent" is a chemical compound useful in the treatment of cancer. Examples of chemotherapeutic agents include alkylating agents such as thiotepa and cyclosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, paplomycin, potfiromycin, puromycin, quelamycin, rodorubicin, streptonigrin, streptozocin, tubercidin, ubenimex, zinostatin, zorubicin; anti-metcibolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testotactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK®; razoxane; sizofiran; spirogermanium; tenuazonic acid; triaziquone; 2, 2',2"-trichtorotriethylamine; urethan; vindesine; dacarbazine; mannomustine; mitobronitol; mitolactol; pipobroman; gacytosine; arabinoside ("Ara-C"); cyclophosphamide; thiotepa; taxanes, e.g. paclitaxet (TAXOL®, Bristol-Myers Squibb Oncology, Princeton, N.J.) and docetaxel (TAXOTERE®; Aventis Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylomithine (DMFO); retinoic acid; esperamicins; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY 117018, onapristone, and toremifene (Fareston); and anti-androgens such as flutamide, nilutamide, bicalutamide, leuprolide, and goserelin; and pharmaceutically acceptable salts, acids or derivatives of any of the above.

A "growth inhibitory agent" when used herein refers to a compound or composition which inhibits growth of a cell, especially a cancer cell either *in vitro* or *in vivo.* Examples of growth inhibitory agents include agents that block cell cycle progression (at a place other than S phrase), such as agents that induce G1 arrest and M-phase arrest. Classical M-phase blockers include the vincas (vincristine and vinblastine), TAXOL®, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C.

### EXAMPLES

The following examples are put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### Example 1: Targeting the Dll4 gene in mice.

Gene Targeting. Velocigene™ technology (Valenzuela et al. (2003) Nat. Biotechnol. 21:652-9) was used to generate a precise deletion and exchange of the Dll4 coding region, extending from the initiation to the termination codon (corresponding an 8.1 kB region comprising all of the coding exons and intervening introns), with the b-galactosidase reporter gene as well as a neomycin selection cassette. Briefly, a bacterial artificial chromosome (BAC) containing the 8.1 kb Dll4 coding region and 140Kb of flanking sequences (clone 475d4 from a 129/SvJ BAC library obtained from Incyte Genomics) was modified to generate a BAC-based targeting vector which was then linearized and used as a targeting vector to replace the Dll4 gene in F1H4 (C57BU6:129 hybrid) mouse embryonic stem (ES) cells. Correctly targeted embryonic stem cells were identified using the loss of native allele (LONA) assay (Valenzuela et al. (2003) *supra*)*.* Two independent correctly targeted ES lines were used to generate chimeric male mice that were complete transmitters of ES-derived sperm. Chimeras were then bred to C57BU6 and/or ICR females to generate F1 mice or embryos, which were genotyped by LONA assays and β-galactosidase histochemical assays. Mice derived from both ES lines behaved identically, and pooled data from both clones were used for statistics.

Results. Targeting the Dll4 gene in mice resulted in embryonic lethality and severe vascular defects, even in mice targeted at a single allele (see Gale et al. (2004) Proc Natl Acad Sci USA 101:15949-15954).

Tumor implantations. Lewis lung carcinoma cells (ATCC) were subcutaneously implanted into the flank of Dll4 chimeric mice, harvested after 16 days, cut into 80 micron sections, and stained for CD31/PECAM or β-galactosidase as described (Holash et al. (2002) Proc Natl. Acad. Sci. USA 99:11393-8).

PECAM and reporter staining. Staining of whole-mounted embryos, as well as tissue sections from embryos and adults, were performed as previously described for CD31/PECAM to define the vascular endothelium and for ß-galactosidase to visualize the Dll4 reporter gene product (Gale et al. (2004) PNAS 101:15949-54).

### Example 2. Dll4-Fc Construct and Mouse Xenograph Studies.

Dll4-Fc (-TM) construct. A nucleic acid sequence was constructed having 2297 nucleotides corresponding to the extracellular domain of mouse Dll4 (SEQ ID NO:3) without the transmembrane (-TM) domain, with a human Fc domain. The encoded amino acid sequence had 765 amino acids including Dll4 protein (SEQ ID NO:18) and a molecular weight of approximately 85 kDa.

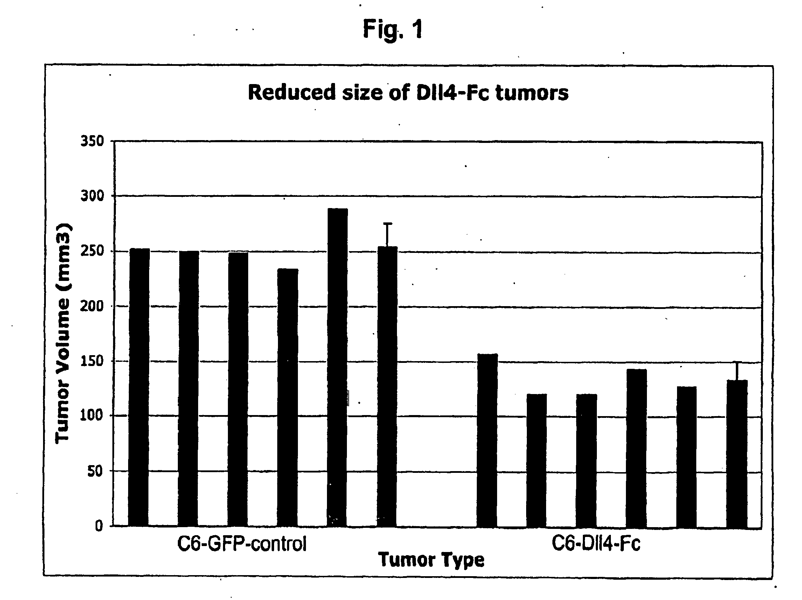

Fig. 1 shows that Dll4-Fc over-expression by C6 tumor cells resulted in smaller C6 tumors (mean ± SD).

Retroviral engineering of tumor cells to over-express Dll4-Fc. C6 rat glioma tumor cells (ATCC) were infected with retrovirus to over-express green fluorescent protein (GFP) and soluble Dll4-Fc; cells infected with GFP alone were used as controls. Cells were FACS sorted for GFP fluorescence twice.

Retrovirus delivered Dll4-Fc. 10⁶ cells/mouse were implanted subcutaneously into the shaved right flank of male SCID/CB17 mice (8-10wok old) with either GFP or Dll4-Fc retrovirally engineered C6 cells.

Tumor volume measurements: After tumors became palpable, size measurements were recorded every three days using a caliper (size= (length x width²)/2). Once animals were sacrificed, *ex vivo* measurements were obtained with calipers and volume was calculated using the formula length x width x height).

Tumor Histology. Twelve to sixteen days after tumor cell implantation, tumors were harvested and processed for histological or expression analysis. Tumors were cut into 80 µm sections, stained with antibodies to CD31/Pecam-1 followed by DAB-peroxidase reaction, and counterstained with pyronin Y. Vessel morphometric analysis was performed using the NIH Image 1.62 analysis program.

Northern Blotting and Real Time-PCR. Total RNA was prepared from tumor tissue using Trizol reagent (Life Technologies, Grand Island, NY). RNA (10 mg) was separated on 1.2% agarose gels, transferred to nylon membrane and immobilized by UV crosslinking. After prehybridization, 32P-labeled Dll4 or glyceraldehyde-3-phosphate dehydrogenase (GAPDH)-specific probes were added, and the filters were hybridized at 42°C overnight. Stringent washes were performed by standard protocols (one wash 0.5 X SSPE buffer followed by two washes with 0.2 X SSPE buffer performed at 55°C for 30 minutes each). An autoradiograph was obtained after 48 h exposure to x-ray film with intensifying screens. In addition, tissue specific expression was analyzed in separate reactions using the Taqman® (Applied Biosystems, Foster City, California) real-time PCR chemistry and detection system with the primers pairs and labeled probes specific for Dll4, the notch receptors 1 and 4 and notch downstream targets, Hes1. Hey2, HeyL and Nrarp. The number of cycles necessary to reach the threshold for amplification of the cDNA (or CT values) was obtained, and normalized to a housekeeping reference (GAPDH) (=2-DCT). The results were normalized to a baseline, the vehicle control for the experiment, giving the relative mRNA abundance change (=2DDCT) and is expressed as the mean ± s.e.m. for at least 4 separate samples run in triplicate (Livak and Schmittgen (2001) Methods. Dec;25(4):402-8).

Quantitative RT-PCR analysis for Dll4, HeyL, Nrarp and Hes1. The RT-PCR analysis was performed as described (Livak et al. (2001) Methods 24:402-8). Results are expressed as the ratio of the amount of the RNA of interest to the amount of control RNA (GAPDH) as described (Daly et al. (2004) Genes Dev. 18:1060-71) on an Applied Biosystems 7900HT using specific primers and probes as follows: Dll4 Primers: Dll4-1574F (SEQ ID NO:9) and Dll4-1644R (SEQ ID NO:10) and Dll4 Probe: Dll4-1594T (SEQ ID NO:11); HeyL Primers: mHeyL-135F (SEQ ID NO:12) and mHeyL-216R (SEQ ID NO:13) and HeyLProbe: mHeyL-154T (SEQ ID NO:14); Nrarp Primers: mNrarp-350F (SEQ ID NO: 15) and mNrarp-418R (SEQ ID NO:16) and Nrarp probe: mNrarp-373T: (SEQ ID NO:17) and mHesl (ID Mm00468601 m1, Hes1) (ABI, Assay on demand services). cDNAs were derived from C6-Dll4-Fc and C6-Dll4 tumors

*In vitro* assay to determine if secreted Dll4-Fc expressed in C6 cells can activate Notch signaling in HUVEC. 4 x 10⁵ HUVEC cells were plated onto 60 mm dish to obtain ∼50% confluent cultures the following day. The next day, 8 x 10⁵ C6 cells were plated on top of HUVECs. After 24 hrs of co-culture, cells were scrapped into 1 ml of Tri Reagent and total RNA was prepared as previously described. Samples were analyzed by Taqman® using human specific Hes1, HeyL and Nrarp probes.

### Example 3. Effect of systemic administration of Dll4-Fc.

Dll4-Fc protein. Plasmid encoding Dll4-Fc cDNA construct described above was transfected into CHO cells, and secreted protein was purified from the supernatant. Dll4-Fc protein was purified and used to treat tumor bearing mice via subcutaneous injection (10 mg/kg, 3x per week).

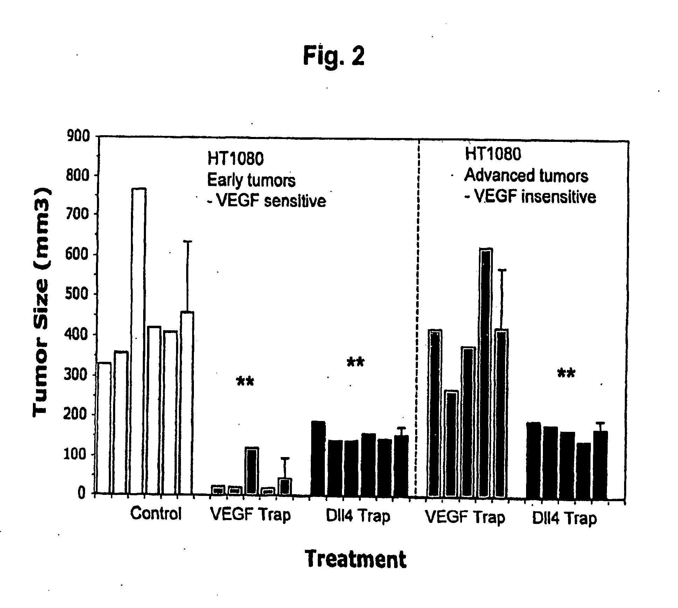

Results. Experiments were conducted in which HT1080 tumors were implanted into mice as described above at day 0. Starting on either day 0 or day 15 (at 100 mm³ in size), mice were treated with purified Dll4-Fc protein (10mg/kg, 3x per wk) or control protein. Other groups were treated with VEGF antagonist (VEGF Trap, SEQ ID NO:19) at a dose of 25 mg/kg, three times per week. The results are shown in Fig. 2. In tumors treated from day 0 (left hand side), both VEGF antagonist and Dll4-Fc were effective at controlling tumor growth. In tumors treated from 100 mm³ in size (right hand side), Dll4-Fc was again effective at controlling tumor growth, and was in fact more effective than VEGF antagonist.

Quantification of circulating Dll4-Fc and hFc. Serum samples obtained from GFP or Dll4-Fc treated mice bearing tumors were analyzed by ELISA assay. ELISA was performed by coating plates with hFc as the capture antibody, blocked with 0.2% I-Block solution (Tropix) and using hFc conjugated to peroxidase as a report antibody. Purified hFc and Dll4-Fc proteins were included as standard curves.

VEGF-Inhibitor treatment. VEGF trap (R1 R2) (Regeneron Pharmaceuticals) (SEQ ID NO:19) or placebo (5% vol/vol PBS/glycerol) was administered subcutaneously to mice bearing 100 mm³ tumors at a dose of 25 mg/kg every three days until the end of the study.

Adenovirus delivery of Dll4-Fc. Other experiments not shown have used adenovirus to deliver Dll4-Fc systemically. C6, HT1080, or MMT tumor cells were implanted subcutaneously into the shaved right flank of male SCID/CB17 mice (8-10wk old). After 24 hours, 1 x 10⁹ pfu of adeno-hFc or adeno-Dll4-Fc was injected into the jugular vein of the mice. Similar results on tumor growth were seen with adeno-Dll4-Fc as with systemic treatment with Dll4-Fc protein.

### Example 4. Effect of polyclonal antibodies to Dll4-Fc on HT1080 tumors.

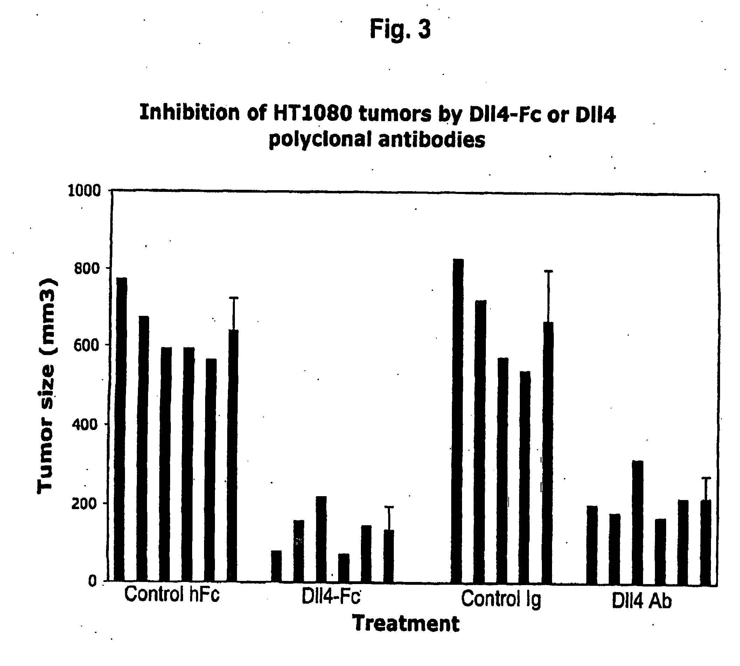

Experiments were conducted in which HT1080 tumors were implanted into mice on day 0 as described above. When the tumors reached 100 mm³ (approximately at day 15), mice were treated three times per week with Dll4-Fc alone (25 mg/kg), control antibody (rabbit lg), or anti-Dll4 polyclonal antibodies depleted for binding to human Fc (10 mg/kg). Results show tumor size in each treatment group ± S.D. (Fig. 3) Dll4 antibodies were highly effective against HT1080 tumor growth and had an effectiveness similar to that seen with Dll4-Fc. These results show that a specific blocker of Dll4 is a potent anti-tumor agent.

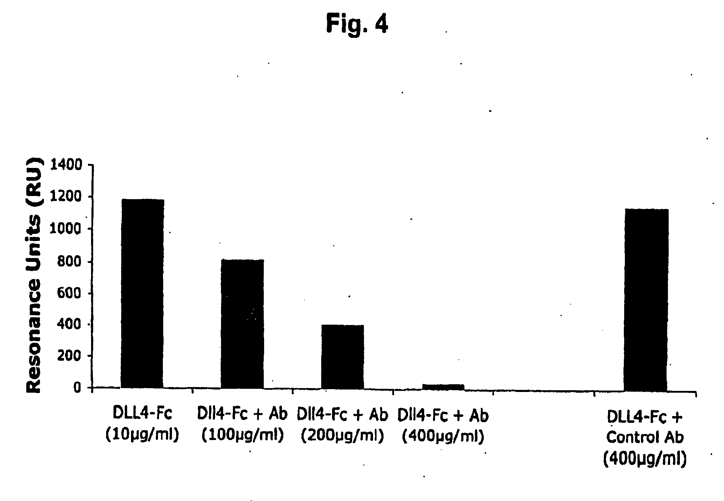

Surface plasmon resonance (BiaCore®) assays were performed confirming the Dll4 antibodies were capable of blocking Dll4 binding to Notch receptor. Notch 1 was coated on the chip surface and Dll4-Fc was incubated with increasing amounts of rabbit polyclonal antii-Dll4 antibody (described above). The results in Fig. 4 show that increasing amount of Dll4 antibody increasingly blocked Dll4-Fc binding to Notch1 (control = Dll4-Fc + non-specific rabbit polyclonal antibody).

## Claims

1. A delta-like ligand 4 (Dll4) antagonist and a vascular endothelial growth factor (VEGF) inhibitor for use in a method for treating tumor development or growth, wherein said Dll4 antagonist is a fusion protein comprising an extracellular domain of Dll4 and a human Fc domain of SEQ ID NO: 20, and wherein the VEGF inhibitor is a VEGF trap of SEQ ID NO: 19.

## Patentansprüche

1. Delta-like-Ligand-4 (Dll4)-Antagonist und ein Gefäßendothelwachstumsfaktor (VEGF)-Inhibitor zur Verwendung in einem Verfahren zur Behandlung von Tumorentwicklung oder -wachstum, wobei der Dll4-Antagonist ein Fusionsprotein ist, das eine extrazelluläre Domäne von Dll4 und eine humane Fc-Domäne von SEQ ID NO: 20 umfasst, und wobei der VEGF-Inhibitor ein VEGF-Trap von SEQ ID NO: 19 ist.

## Revendications

1. Un antagoniste de ligand Delta-like 4 (Dll4) et un inhibiteur de facteur de croissance de l'endothélium vasculaire (VEGF) pour utilisation dans un procédé de traitement du développement ou de la croissance d'une tumeur, lequel antagoniste de ligand Dll4 est une protéine de fusion comprenant un domaine extracellulaire de ligand Dll4 et un domaine de fragment Fc humain de Séquence N° 20 et lequel inhibiteur de facteur VEGF est un piège à VEGF de Séquence N° 19.
